(19) **Europäisches Patentamt / European Patent Office / Office européen des brevets**

(11) **EP 2 637 706 B1**

(12) **EUROPEAN PATENT SPECIFICATION**

(45) Date of publication and mention
of the grant of the patent:
**18.03.2015 Bulletin 2015/12**

(51) Int Cl.:
**A61F 13/49** (2006.01) **A61L 15/42** (2006.01)
**A61L 15/24** (2006.01)

(21) Application number: **11791673.4**

(86) International application number:
**PCT/US2011/060079**

(22) Date of filing: **10.11.2011**

(87) International publication number:
**WO 2012/064909 (18.05.2012 Gazette 2012/20)**

(54) **ELASTOMERIC COMPOSITIONS THAT RESIST DISINTEGRATION**

ZERFALLBESTÄNDIGE ELASTOMERE ZUSAMMENSETZUNGEN

COMPOSITIONS ÉLASTOMÈRES QUI RÉSISTENT À LA DÉSINTÉGRATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.11.2010 US 412847 P**

(43) Date of publication of application:
**18.09.2013 Bulletin 2013/38**

(73) Proprietor: **The Procter & Gamble Company
Cincinnati, OH 45202 (US)**

(72) Inventors:
• **MELIK, David, Harry
Cincinnati
Ohio 45252 (US)**

• **SMITH, Steven, Daryl
Fairfield
Ohio 45014 (US)**
• **NETON, Janet
West Chester
Ohio 45069 (US)**

(74) Representative: **L'Huillier, Florent Charles et al
Procter & Gamble Service GmbH
IP Department
Frankfurter Strasse 145
61476 Kronberg im Taunus (DE)**

(56) References cited:
**WO-A1-2009/066268    US-A1- 2005 273 071
US-A1- 2005 273 072**

**Description**

FIELD OF THE INVENTION

This invention is directed to absorbent articles such as diapers, training pants, adult incontinence articles, feminine hygiene articles, and the like comprising a stretch laminate.

BACKGROUND OF THE INVENTION

It may be desirable to construct absorptive devices, such as disposable diapers with fasteners, pull-on diapers, training pants, sanitary napkins, pantiliners, incontinence briefs, and the like, with stretch laminates to improve the ease of motion and maintenance of a sustained fit. Furthermore, stretch laminates allow the diaper to accommodate a range of different sized wearers. A diaper may have stretch laminates in a number of its article elements including the waist band, leg cuffs, side panels, elasticized topsheets, backsheet, ears, outercover, and fastening system.

As disclosed in U.S. Pat. No. 7,717,893 and U.S. Pub. No. US 2005-0273071, there is a need for an absorbent product comprising a stretch laminate that retracts slowly upon being released from a stretched state, thus facilitating application and positioning of the product correctly onto the wearer. U.S. Pat. No. 7,717,893 and U.S. Pub. No. US 2005-0273071 further disclose various embodiments of slow recovery polymers, films, and/or laminates for the purpose of meeting said need, as well as meeting various other needs and desires.

A problem that can exist in filling the need for a stretch laminate is that during the use of an absorbent article comprising a stretch laminate comprising an elastic member, certain baby oils, lotions, gels, cremes, and the like, that are spread on the wearers skin before application of the article, may be absorbed to some extent by the elastic member. This absorption may lead to a swelling or breakage of the elastic member and may result in reduced performance. Swelling may lead to (1) a sticky feeling stretch laminate that may cause discomfort to the wearer of the absorbent article, and/or (2) a reduction in the unload force of the stretch laminate at 37°C, which may result in poor fit and may lead to, for example, increased urine or bowel movement leakage during use, sagging or drooping of the absorbent article during use, and/or increased discomfort in wearing the absorbent article. Breakage of the elastic member may lead to poor fit if it is localized, but if widespread may lead to catastrophic failure of the stretch laminate which may lead to the failure of the absorbent article comprising the stretch laminate. The degree to which absorption may occur may depend on the particular construction of the stretch laminate, for example, on the type and basis weight of the substrate and on the type and basis weight of any adhesive used to join the elastic member to the substrate, as well as where it is located within the absorbent article. For example, the use of a stretch laminate as a waist feature or side panel is in an area of an absorbent article that is less likely to encounter residual baby oil, lotions, gels, and the like, as compared to the use of the stretch laminate as an elasticized topsheet.

It is an object of the present disclosure to provide various embodiments that offer solutions to said problem, while still also meeting the needs and desires of using a slow recovery polymer, film, and/or laminate as disclosed in U.S. Pat. No. 7,717,893 and U.S. Pub. Nos. 2005-0273071, 2006-0155255, 2006-0167434, and 2009-0134049.

Further, it is an object of the present disclosure to provide various embodiments for both slow and conventional recovery elastomers, films, and laminates comprising an hydrogenated block copolymer useful for overcoming the problem expressed in the previous paragraph. Still further, it is an object of the present disclosure to provide various properties of both slow and conventional recovery polymers, films, and laminates that in combination with an hydrogenated block copolymer are useful for overcoming the problem expressed in the previous paragraph including (1) aromatic substitution of either or both the soft block and the hard block, (2) hard blocks with a solubility parameter of greater than 9.1 $(cal/cm^3)^{1/2}$, (3) compositions that remain extendable to at least 50% engineering strain after exposure to isopropyl palmitate for 30 hours at room temperature, and (4) certain combinations thereof.

SUMMARY OF THE INVENTION

In response to the problem identified above, the present disclosure provides an embodiment of an absorbent article comprising a topsheet, a backsheet joined with the topsheet, an absorbent core interposed between the topsheet and backsheet; and an article element as defined in the claims. The article element comprises a stretch laminate comprising an elastic member comprising a block copolymer comprising at least one soft block and at least two hard blocks, wherein the soft block backbone is hydrogenated. Further, the hydrogenated block copolymer comprises one or both of the following: (a) one or more hard blocks comprising substituted polystyrene; (b) one or more soft blocks comprising substituted polystyrene.

Further, the present disclosure provides an embodiment for a slow recovery stretch laminate exhibiting an unload force at 37°C of about 0.16 N/(g/m) or greater and a percent of initial strain after 15 seconds of recovery at 22°C of about 10% or greater.

BRIEF DESCRIPTION OF THE DRAWINGS

FIGS. 1A-E are perspective views of embodiments of the stretch laminate.

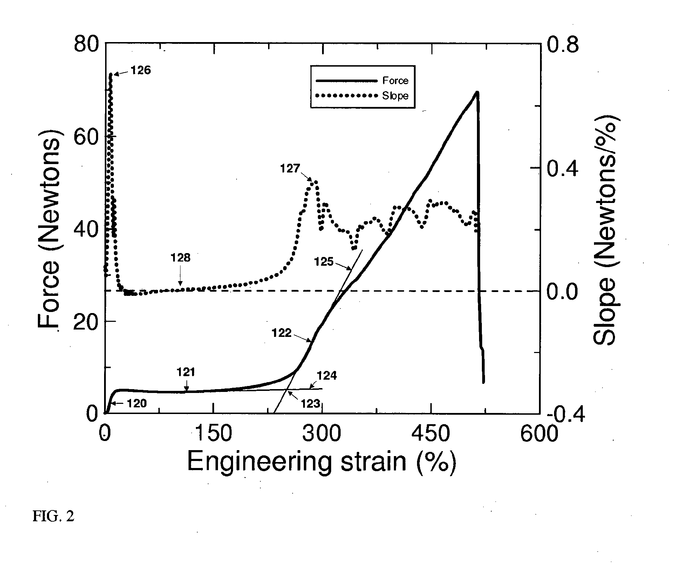

FIG. 2 shows a representative force-engineering strain curve for a stretch laminate embodiment and illustrates the approach for determining the maximum laminate strain.
FIG. 3a shows a representative structure of a polystyrene block copolymer with an isoprene soft block.
FIG. 3b shows a representative structure of a polystyrene block copolymer with an ethylene/propylene soft block.
FIG. 3c shows a representative structure of a polystyrene block copolymer with an ethylene/propylene soft block and a nitrated hard block where the degree of nitration depicted is 100%.

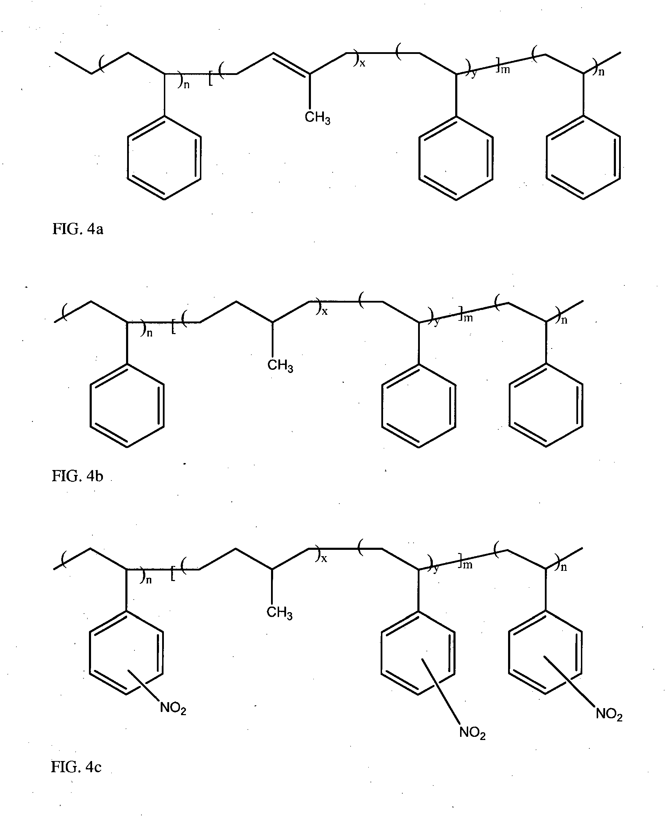

FIG. 4a shows a representative structure of a polystyrene block copolymer with a random styrene-isoprene soft block.
FIG. 4b shows a representative structure of a polystyrene block copolymer with an random styrene-ethylene/propylene soft block.
FIG. 4c shows a representative structure of a polystyrene block copolymer with a random nitrated styrene-ethylene/propylene soft block and a nitrated hard block where the degree of nitration depicted is 100%.

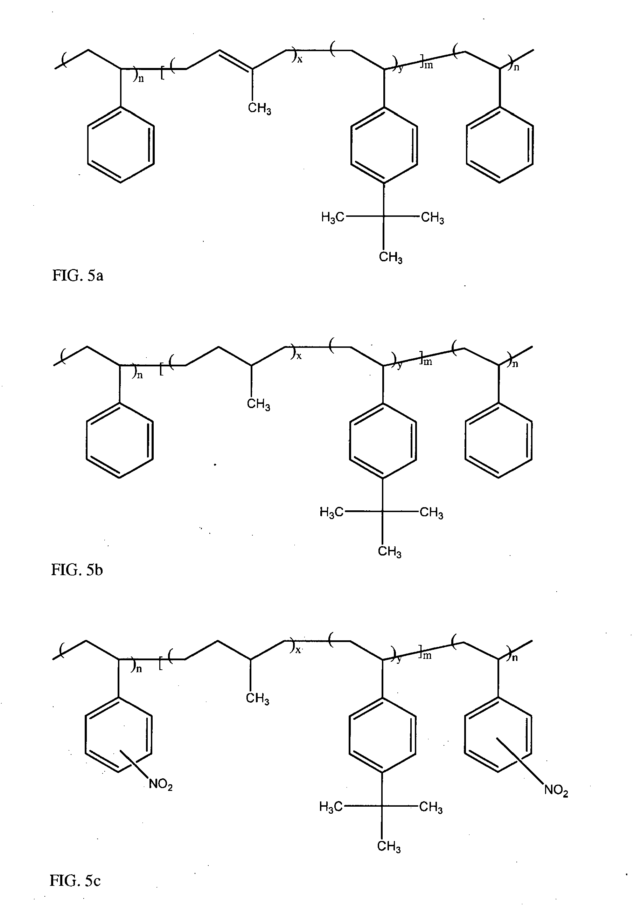

FIG. 5a shows a representative structure of a polystyrene block copolymer with a random t-butyl styrene-isoprene soft block.
FIG. 5b shows a representative structure of a polystyrene block copolymer with an random t-butyl styrene-ethylene/propylene soft block.
FIG. 5c shows a representative structure of a polystyrene block copolymer with a random t-butyl styrene-ethylene/propylene soft block and a nitrated hard block where the degree of nitration depicted is 100%.

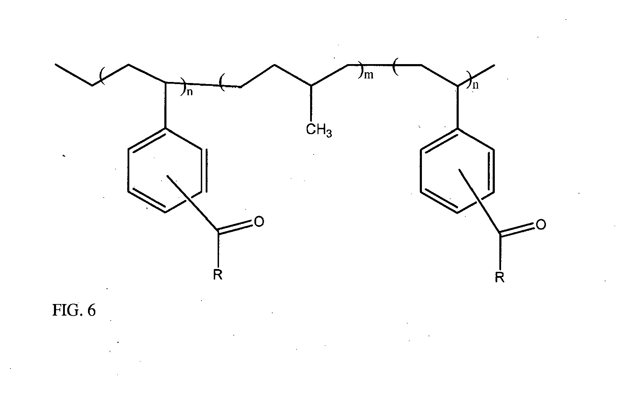

FIG. 6 shows a representative structure of a polystyrene block copolymer with an ethylene/propylene soft block and a ketone substituted hard block where the degree of substitution depicted is 100%.

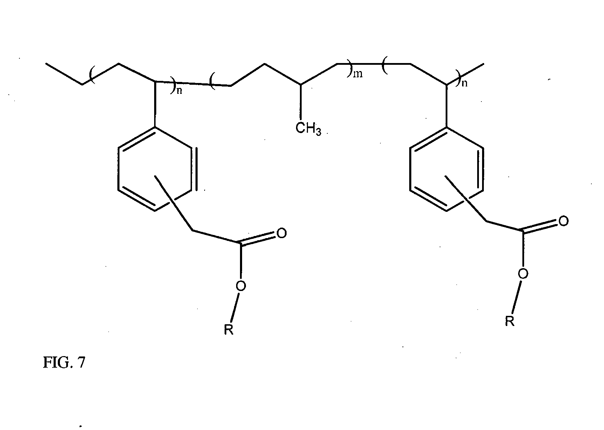

FIG. 7 shows a representative structure of a polystyrene block copolymer with an ethylene/propylene soft block and an acetyl ester substituted hard block where the degree of substitution depicted is 100%.

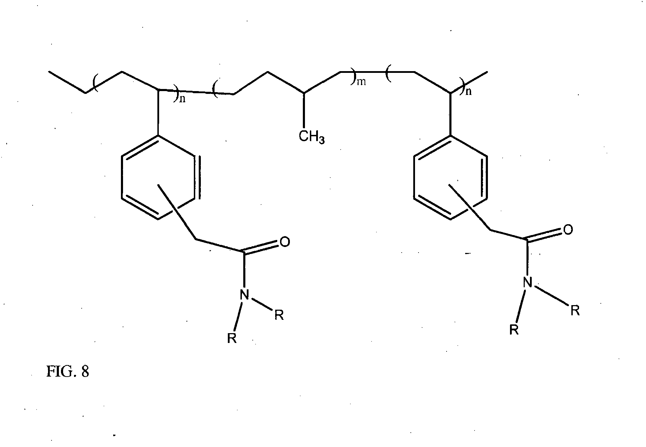

FIG. 8 shows a representative structure of a polystyrene block copolymer with an ethylene/propylene soft block and an amide substituted hard block where the degree of substitution depicted is 100%.

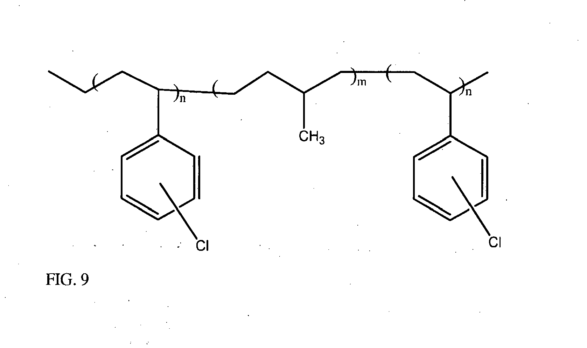

FIG. 9 shows a representative structure of a polystyrene block copolymer with an ethylene/propylene soft block and a chlorine substituted hard block where the degree of substitution depicted is 100%.

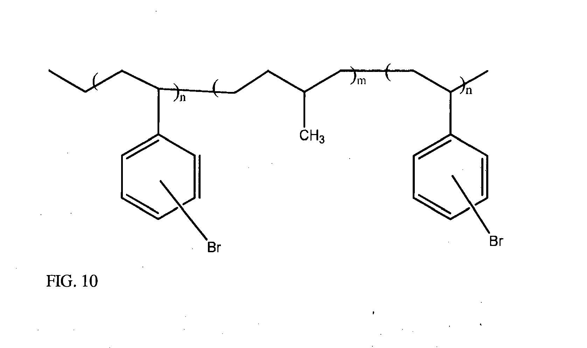

FIG. 10 shows a representative structure of a polystyrene block copolymer with an ethylene/propylene soft block and a bromine substituted hard block where the degree of substitution depicted is 100%.

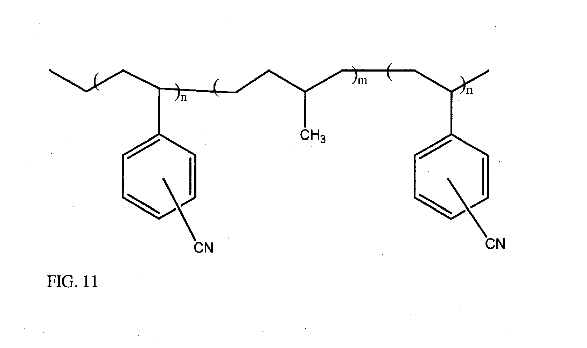

FIG. 11 shows a representative structure of a polystyrene block copolymer with an ethylene/propylene soft block and a nitrile substituted hard block where the degree of substitution depicted is 100%.

DETAILED DESCRIPTION OF THE PRESENT DISCLOSURE

As used herein, the term "absorbent article" or "article" refers to a wearable device that absorbs and/or contains liquid and, more specifically, refers to a device that is placed against or in proximity to the body of the wearer to absorb and contain the various exudates discharged from the body. Suitable examples include diapers, training pants, pull-on garments, adult incontinence products and feminine care products such as sanitary napkins. Furthermore, "absorbent article" includes "disposable absorbent article" which is intended to be discarded and not laundered or otherwise restored after no more than ten uses (although certain components may be recycled, reused, or composted).

As used herein, the term "stretch laminate" generally refers to an elastomer which is attached to at least one material such as a polymeric film, a nonwoven, a woven, or a scrim. The elastomer may be attached to the material by any of a number of bonding methods known to those skilled in the art, including adhesive bonding, thermal bonding, pressure bonding, ultrasonic bonding, and the like, or any combination thereof.

As used herein, the term'laminate"refers to a material comprising two or more layers. The term includes stretch laminates and non-stretch laminates.

As used herein, the term "diaper" refers to an absorbent article generally worn by infants and incontinent persons about the lower torso.

As used herein, the term"substrate"refers to a material that is laminated to the elastic member to form the stretch laminate. Suitable substrates include nonwoven webs, woven webs, knitted fabrics, films, film laminates, apertured films, nonwoven laminates, sponges, foams, scrims, and any combinations thereof. Suitable substrates may comprise natural materials, synthetic materials, or any combination thereof.

As used herein, the term "longitudinal" generally means a direction running parallel to the longitudinal axis, of the article and includes directions within 45° of the longitudinal direction.

As used herein, the term "length" of the article or component thereof generally refers to the size/distance of the maximum linear dimension, or the size/distance of the longitudinal axis, or an article or part thereof.

As used herein, the terms "lateral" or "transverse" refer to a direction generally orthogonal to the longitudinal direction and parallel to the transverse axis.

As used herein, the term "width" of the article or of a component thereof refers to the size/distance of the dimension orthogonal to the longitudinal direction of the article or component thereof, *e.g.*, orthogonal to the length of the article or component thereof, and may refer to the distance/size of the dimension parallel to the transverse axis of the article or component.

As used herein, the term "attached" encompasses configurations whereby an element is directly secured to another element by affixing the element directly to the other element.

As used herein, the term "joined" or "connected" encompasses configurations whereby a first element is directly secured to second element by affixing the first element directly to the second element and configurations whereby a first element is indirectly secured to a second element by affixing the first element to intermediate member(s), which in turn are affixed to the second element. "Joined'or"connected'elements may be affixed either continuously or intermittently.

As used herein, "relaxed" or "relaxed state" means the state where no forces are applied to an article (other than naturally occurring forces such as gravity).

As used herein, the terms "extendibility" and "extensible", *e.g.*, extendibility of the elastomer, mean that the width or length of the item in the relaxed position can be extended or increased.

As used herein, "elasticated" or "elasticized" means that the component comprises at least a portion made of elastic material.

As used herein, the terms "elastic," "elastomer," and "elastomeric" refer to a material which generally is able to extend to a strain of at least 50% without breaking or rupturing, and is able to recover substantially to its original dimensions after the deforming force has been removed.

As used herein, the term "medical product" means surgical gowns and drapes, face masks, head coverings, shoe coverings, wound dressings, bandages and sterilization wraps as disclosed in U. S. Patent No. 5,540,976.

As used herein, the term "copolymer" refers to a polymer synthesized from two or more monomers with different chemical structures.

As used herein, the terms "temperature responsive" and "temperature responsiveness" refer to a slow recovery stretch laminate exhibiting less post elongation strain after a specified amount of time at higher temperatures than at lower temperatures.

As used herein, the term "conventional stretch laminate" refers to a stretch laminate that exhibits a minimal percent of initial strain after 15 seconds of recovery at 22°C as measured by the Post Elongation Recovery Test. Conventional stretch laminates exhibit a percent of initial strain after 15 seconds of recovery at 22°C of less than 10%, as measured by the Post Elongation Recovery Test.

As used herein, the term "percent of initial strain remaining" refers to the percentage of initial strain remaining after some period of time after release from that initial strain as measured by the Post Elongation Recovery Test. "Percent of initial strain remaining" is calculated by dividing the percent strain at a given time after release from an initial strain by the initial percent strain; the quotient is multiplied by 100 to yield a percentage.

As used herein, the terms "stress-engineering stress" and "nominal stress" refer to the load divided by the initial undeformed cross-sectional area of the sample on which a deformation force acts.

As used herein, the terms "strain" and "engineering strain" refer to the change in sample length divided by the initial undeformed length of the sample on which a deformation force acts, usually expressed as a percent.

As used herein, the tern "yield point" refers to the point on an engineering stress versus strain curve beyond which deformation is not completely recoverable, the term "yield stress" refers to the engineering stress value at the yield point, and the term'yield strain" refers to the level of strain at the yield point usually expressed as a percent strain. Some materials may also exhibit a "yield drop" i.e., a decrease in engineering stress with increasing strain. As used herein, the terms "strain at break, "strain at failure" and "ultimate strain" refer to the maximum tensile strain to which a material can be subjected before it breaks, and may be expressed as the percentage strain.

As used herein, the term "hard block" refers to the block or blocks in a block copolymer that have a glass transition temperature (or melt temperature if the block is crystallizable) above use temperature.

As used herein, the term "hard phase" refers to the phase or phases in a block copolymer composition that are uniform in chemical composition and physical state and that have a glass transition temperature (or melt temperature if the block is crystallizable) above use temperature. The hard phase may comprise multiple hard blocks of a block copolymer and any hard block associating ingredients including, but not limited to, processing oils and modifying resins.

As used herein, the term "soft block" refers to the block or blocks in a block copolymer that have a glass transition temperature (or melt temperature if the block is crystallizable) below use temperature.

As used herein, the term "soft phase" refers to the phase or phases in a block copolymer composition that are uniform in chemical composition and physical state and that have a glass transition temperature (or melt temperature if the block is crystallizable) below use temperature. The soft phase may comprise multiple soft blocks of a block copolymer and any soft block associating ingredients including, but not limited to, processing oils and modifying resins.

As used herein, the term "block copolymer composition" refers to a polymer blend comprising a block copolymer.

As used herein, the term "morphology" refers to the shape, appearance, or form of phase domains in substances, such as polymers, polymer blends, composites, and crystals. The morphology describes the structures and shapes observed, including by microscopy or scattering techniques, of the different phase domains present within the substance. For example, for block copolymers and block copolymer compositions, common morphologies include spherical and cylindrical such as described in Thermoplastic Elastomers, 2(nd) Edition, Chapters 3, 4, 11, and 12, G. Holden, et al. (Editors), Hanser Publishers, New York (1996).

As used herein, the term "phase domain" or "phase" refers to the region of a material that is uniform in chemical composition and physical state. In a multiphase material, each phase may form domains differing in size, chemical composition, or physical state from the other phases. For example, block copolymer compositions may comprise soft phase and hard phase regions that are uniform in chemical composition and physical state within each phase region, but the hard and soft phases generally differ from each other in size, chemical composition, and physical state. Further, For example, semi-crystalline homopolymers like polypropylene may comprise crystalline and amorphous phases differing only in size and physical state.

As used herein, the term "hysteresis" refers to the dissipation of energy in a cyclic process. For example, in a tensile hysteresis experiment where the stress is plotted against strain as the strain is increased to a target strain less than the strain at break in a load cycle, followed by decreasing the strain in an unload cycle, the two curves do not coincide but form a hysteresis loop with the unload cycle having a lower stress at a given strain compared to the load cycle.

As used herein, the term "tensile modulus of elasticity" or 'Young's modulus" is a measure of the stiffness of a material. For thin materials (less than about 1.0 millimeter in thickness), the tensile modulus of elasticity can be determined according to ASTM D 882; while for thick materials (greater that about 1.0 millimeter and less than about 14 millimeters in thickness), the tensile modulus of elasticity can be determined according to ASTM D 638.

As used herein, the term "EHB polymer" or "Equivalent Hard Block polymer" refers to a polymer having essentially the same repeat unit composition and composition distribution as a hard block of a block copolymer composition. For example, for a block copolymer composition comprising a styrene-ethylene/propylene-styrene (SEPS) block copolymer, an equivalent hard block polymer is polystyrene. Additionally, a block copolymer composition may be characterized by more than one equivalent hard block polymer. For example, for a block copolymer composition comprising a SEPS block copolymer and an alphamethylstyrene- ethylene/propylene-alphamethylstyrene block copolymer, the EHB polymers for this composition would be polystyrene and poly(alphamethylstyrene). In certain embodiments of the present disclosure, the ratio of the number average molecular weight of each EHB polymer to the number average molecular weight of its corresponding hard block in the block copolymer composition may range from about 0.5 to about 100, or may range from about 1 to about 50, or may range from about 1 to about 20.

As used herein, the term "ESB polymer" or "Equivalent Soft Block polymer" refers to a polymer having essentially the same repeat unit composition and composition distribution as a soft block of a block copolymer composition. For example, for a block copolymer composition comprising a styrene-ethylene/propylene-styrene (SEPS) block copolymer, an equivalent soft block polymer is poly (ethylene/propylene). Additionally, a block copolymer composition may be characterized by more than one equivalent soft block polymer. For example, for a block copolymer composition comprising a SEPS block copolymer and a styrene-ethylene/butylene-styrene (SEBS) block copolymer, the ESB polymers for this composition would be poly(ethylene/propylene) and poly(ethylene/butylene). In certain embodiments of the present disclosure, the number average molecular weight of each ESB polymer may range from about 0.3 to about 150 kilo Daltons.

As used herein, the term 'hard block associating" or "chard phase associating" refers to ingredients of a block copolymer composition that phase mix with the hard block of the block copolymer. Generally, hard block or hard phase associating ingredients have a solubility parameter similar to the solubility parameter of the hard block. In certain embodiments of the present disclosure, the solubility parameter of the hard block or hard phase associating ingredients may be within about 0.5 $(cal/cm^3)^{1/2}$ of the solubility parameter of the hard block. Further, in certain embodiments of the present disclosure, the hard block or hard phase associating ingredients may raise or lower the glass transition temperature of the hard phase from its value in the pure block copolymer. Examples of hard block associating ingredients include, but are not limited to, modifying resins, processing oils, and hard phase modifiers.

As used herein, the term "skin layet" refers to an outer layer of a coextruded, multilayer film that acts as an outer surface of the film during its production and subsequent processing.

Embodiments of absorbent articles of the present disclosure may comprise a slow recovery stretch laminate (SRSL). The SRSL may be used within the absorbent article wherever elastic properties are desired. The SRSL may comprise an elastic member joined to a substrate. The SRSL may be formed discretely and joined with the absorbent article. Conversely, the SRSL may be integral to the absorbent article (*e.g.*, an elastic member is joined to an existing substrate in the absorbent article such as the topsheet to form a stretch laminate). The elastic member may be prepared from a composition comprising an elastomeric polymer, optionally at least one modifying resin, and optionally one or more additives. The SRSL may exhibit a normalized unload force at 37°C of at least about 0.16 N/(g/m) as measured by the Two Cycle Hysteresis Test described below. The SRSL may exhibit a percent of initial strain after 15 seconds of recovery at 22°C of about 10% or greater, as measured by the Post Elongation Recovery Test as described below.

In another embodiment of the present disclosure, the SRSL may be incorporated into a medical product such as a surgical gown, a face mask, a head covering, a shoe covering, a wound dressing, a bandage, or a sterilization wrap. The SRSL may be used in the medical products at locations where an elastic character is desired.

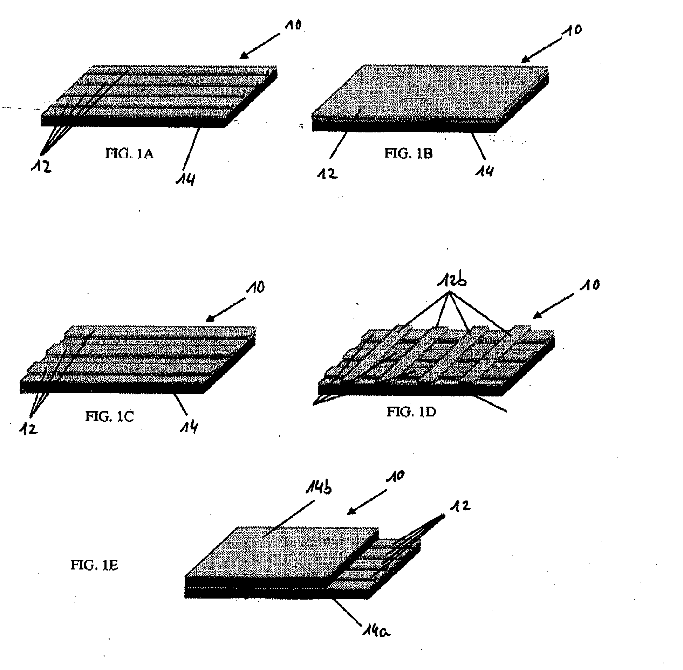

As shown in FIGS. 1A-E, the stretch laminate 10, which can be either a slow recovery stretch laminate, a conventional stretch laminate, or a combination thereof, may comprise an elastic member 12 joined to a substrate 14. Joining of the elastic member 12 and the substrate 14 may be conducted by a variety of bonding methods such as heat bonds, pressure bonds, ultrasonic bonds, mechanical bonds, adhesive bonds, or any other suitable attachment means or combinations of these attachment means. In certain embodiments, the elastic member 12 may exhibit sufficient tack to join the elastic member 12 and the substrate 14.

The elastic members 12 having a variety of forms may be used in the stretch laminate 10. Suitable forms for the elastic members 12 include, but are not limited to films, bands, strands, individualized fibers, scrims, cross-hatch arrays, foams, or combinations thereof.

FIGS. 1A-E depict several suitable embodiments of the stretch laminate 10. FIG. 1A depicts a stretch laminate 10 having one or more elastic members 12 in the form of bands or ribbons joined with a substrate 14. FIG. 1B depicts a stretch laminate 10 having a sheet-like elastic member 12 joined with a sheet-like substrate 14. The elastic member 12 and the substrate 14 are shown as being coterminous; however, either layer may have dimensions differing from the other layer. FIG. 1C depicts a stretch laminate 10 having one or more elastic members 12 in the form of strands joined with a substrate 14.

FIG. ID depicts a stretch laminate 10 having one or more elastic members in the form of a cross-hatch array joined with a substrate 14. A cross-hatch array may be formed in one instance by joining a plurality of elastic members 12a in parallel to the substrate 14. A second plurality of elastic members 12b may be joined in parallel to the substrate. The second plurality 12b may be joined in a non-parallel configuration to the first plurality 12a. A cross-hatch array may also be formed by hot needle punching of an elastomeric film. A cross-hatch array may also be formed from a porous, macroscopically-expanded, three-dimensional elastomeric web as described in U.S. Patent Application Publication No. 2004/0013852. The publication describes how the cross-hatch array can be achieved by forming the film on a porous forming structure and applying a fluid pressure differential across the thickness of the film. The fluid pressure differential causes the film to conform to the supporting structure and rupture thereby creating a cross-hatch array. FIG. 1E depicts a stretch laminate 10 having one or more elastic members 12 joined to two or more substrates: first substrate 14a and second substrate 14b. The particular order of the stretch laminate 10 layers can vary; however, in the embodiment depicted, the elastic members 12 are disposed between the first substrate 14a and the second substrate 14b, and may be bonded to one or both. The first and second substrate 14a, 14b may comprise the same material or may be distinct.

The techniques for the formation of stretch laminates as disclosed in U.S. Pat. No. 7,717,893 and U.S. Pub. Nos. 2005-0273071, 2006-0155255, 2006-0167434, and 2009-0134049 may be applicable in the formation of the SRSL 10 of the present disclosure. One technique for creating a stretch laminate, which is commonly known as "stretch bonding," involves an elastic member such as elastic strands, bands, ribbons, films, or the like being joined to a substrate while the elastic member is in a stretched configuration. The elastic member may be stretched to at least 25% of its relaxed length. After joining, the elastic member is allowed to relax thereby gathering the substrate and creating a stretch laminate.

Another technique for creating a stretch laminate, which is commonly known as "neck bonding," involves an elastic member being bonded to a substrate while the substrate is extended and necked. In certain embodiments, the substrate may be a non-elastic substrate. Examples of neck-bonded laminates are described in U.S. Pat. Nos. 5,226,992; 4,981,747; 4,965,122; and 5,336,545. A variant of "neck bonding" is "neck stretch bonding." Neck stretch bonding refers to an elastic member being bonded to a substrate while the substrate is extended and necked and the elastic member is extended. Examples of necked stretch bonded laminates are described in U.S. Pat. Nos. 5,114,781 and 5,116,662.

In another technique for forming a stretch laminate, elastic members can be attached to a substrate in either a relaxed configuration or partially stretched configuration. The resulting laminate can be made stretchable (or more stretchable in the case of partially stretched strands or film) by subjecting the laminate to an elongation process which elongates the substrate permanently, but elongates the elastic members only temporarily. Such processes are known in the art as "zero strain" stretch laminate formation, and the elongation of such laminates may be accomplished with suitable means such as rollers, engaging teeth, or the like. Examples of zero strain activation processing and formations of resulting stretch laminates are described in U.S. Patent Nos. 5,167,897 and 5,156,793.

Another technique for the formation of a stretch laminate is disclosed in U.S. Patent Application Publication Nos. 2003/0088228A1, 2003/0091807A1, and 2004/0222553A1. The technique disclosed in these publications involves forming the elastic member by hot melt application of one or more thermoplastic elastomers onto a substrate, followed by incremental stretching of the substrate that confers the stretch properties of the elastomer to the substrate. Suitable application methods include, for example, direct gravure, offset gravure, and flexographic printing. Each of these methods allows deposition of an amount of elastomer in any shape and direction, thus providing substantial flexibility in the stretch character exhibited by the stretch laminate. Other conventional methods for stretch laminate formation are within the scope of this description.

Additionally, to produce reliable stretch laminates, it is important to achieve a relatively uniform strain profile throughout the stretch zone. Materials exhibiting a yield drop may have stability problems during stretching, such as variations in thickness, and thereby generally result in laminates with a high level of property variation. Increasing the stretching temperature, decreasing the strain rate, and/or prestretching the elastic member can make the yield drop less pronounced, thereby improving the chances for achieving a more uniform strain profile. Such results in the fabrication of more reliable stretch laminates. Production of reliable SRSLs may be found in U.S. Pub. No. 2005-0273071.

The elastic member 12 may comprise an elastomeric polymer, optionally at least one modifying resin, and optionally one or more additives. A number of elastomeric polymers, either alone or in combination, can be used to prepare the elastic member 12. Elastomeric polymers include, but are not limited to, homopolymers (*e.g.*, cross-linked poly(isoprene)), block copolymers, random copolymers, alternating copolymers, and graft copolymers. Suitable elastomeric polymers comprise styrenic block copolymers, natural and synthetic rubbers, polyisoprene, neoprene, polyurethanes, silicone rubbers, hydrocarbon elastomers, ionomers, and the like. Other suitable block copolymers include, but are not limited to, polyolefin based block copolymers such as described in Thermoplastic Elastomers, 2nd Edition, Chapter 5, G. Holden, et al. (Editors), Hanser Publishers, New York (1996)

In one embodiment, the elastomeric polymer may be a block copolymer. A number of block copolymers may be used including multi-block, tapered block and star block copolymers. Block copolymers suitable for use in embodiments of the present disclosure may exhibit both elastomeric and thermoplastic characteristics. In such block copolymers a hard block (or segment) may have a glass transition temperature (Tg) greater than about 25°C or is crystalline or semicrystalline with a melting temperature (Tm) above about 25 °C. The hard block may have a Tg greater than about 35 °C or is crystalline or semicrystalline with a Tm above about 35 °C. The hard block portion may be derived from vinyl monomers including vinyl arenes such as styrene and alpha-methyl-styrene, methacrylates, acrylates, acrylamides, methacrylamides, polyolefins including polypropylene and polyethylene, or combinations thereof. For hard blocks comprising substantially polystyrene, the hard block molecular weight may range from about 4 to about 20 kilo Daltons, or may range from about 6 to about 16 kilo Daltons, or may range from about 7 to about 14 kilo Daltons, or may range from about 8 to about 12 kilo Daltons. The weight percent of the hard block in the pure block copolymer may range from about 10 to about 40 weight percent, or may range from about 20 to about 40 weight percent, or may range from about 25 to about 35 weight percent. Further, the stretch laminate embodiments of the present disclosure may comprise a block copolymer that comprises at least two hard blocks. It is possible that block copolymers comprising only one hard block may result in block copolymer compositions that behave more like a viscous liquid than an elastomer. Further, it is possible that block copolymers comprising at least two hard blocks may result in block copolymer compositions that behave like an elastomer.

Glass transition temperatures referred to herein are determined by tensile dynamic mechanical analysis performed in the linear elastic region of the material at a frequency of 1 Hz using a temperature ramp method such as described in ASTM D 5026. Suitably, film samples with a uniform thickness of about 0.3 mm may be used with a temperature ramp rate of about 1 °C/min or slower. The loss modulus peak temperature is taken as the Tg of a particular material or phase..

Crystalline melting temperatures referred to herein are determined by Differential Scanning Calorimetry using a temperature ramp rate of 10°C/min. The melting endothermic peak temperature on the second heat is taken as the Tm of the particular crystalline region, such as described in ASTM D 3418.

The block copolymers may comprise a soft block (or segment). The soft block may exhibit a sufficiently low glass transition temperature and/or melting temperature so as not to form glassy or crystalline regions at the use temperature of the copolymer. In one embodiment, the use temperature may be between about room temperature (about 22°C) and about body temperature (about 37°C). However, other use temperatures are feasible and within the scope of this invention. Such soft blocks are generally physically incompatible with the hard blocks and form separate regions, domains, or phases. The stretch laminate embodiments of the present disclosure may comprise a block copolymer that comprises at least one soft block. It is possible that block copolymers comprising at least one soft block may result in block copolymer compositions that behave like an elastomer.

The soft block portion may be a polymer derived from conjugated aliphatic diene monomers. The monomers used to synthesize the soft block may contain fewer than about 6 carbon atoms. Suitable diene monomers include butadiene, isoprene, and the like. Further, it is envisioned that the soft block may be modified to tailor the Tg of the soft block. For example, a random copolymer of styrene and dienes, where the Tg of the soft block may be controlled by the ratio of styrene to diene may be used. Additionally, for example, high 1,2 diene polymers known to have high glass transition temperatures may be used or a graft of styrene onto poly(isoprene) may be used. In such cases, lower amounts of the modifying resin may be used. Additionally, such tailored soft blocks may be hydrogenated. Further, in certain embodiments of the present disclosure, the soft block portion may be a polymer derived from acrylates, silicones, polyesters and polyethers including, but not limited to, poly(ethylene adipate) glycol, poly(butylene-1,4 adipate) glycol, poly(ethylene butylene-1,4 adipate) glycol, poly(hexamethylene 2,2-dimethylpropylene adipate) glycol, polycaprolactone glycol, poly(diethylene glycol adipate) glycol, poly(1,6-hexanediol carbonate) glycol, poly(oxypropylene) glycol, and poly(oxytetramethylene) glycol.

Suitable block copolymers for use in embodiments of the present disclosure may comprise at least two hard blocks (A) and at least one soft block (B). The block copolymers may have multiple blocks. In one embodiment, the block copolymer may be an A-B-A triblock copolymer, an.A-B-A-B tetrablock copolymer, or an A-B-A-B-A pentablock copolymer. Also, useful herein are triblock copolymers having hard blocks A and A', wherein A and A' may be derived from different vinyl compounds. Also, useful in embodiments of the present disclosure are block copolymers having more than one hard block and/or more than one soft block, wherein each hard block may be derived from the same or different monomers and each soft block may be derived from the same or different monomers. It should be noted that where the copolymer contains residual olefinic double bonds, the copolymer may be partially or fully hydrogenated if desired. Saturation may yield beneficial effects in the elastomeric properties of the copolymer.

Suitable star block copolymers may be comprised of multiple arms connected at the center and whose arms are constituted of at least one soft and at least one hard block where the hard block is chosen from either a) blocks of monoalkenyl aromatic hydrocarbons or b) blocks of random copolymers of monoalkenyl aromatic hydrocarbons and conjugated diolefin monomers with glass transition temperatures above 20°C, and the soft block is chosen from either c) blocks of random copolymers of monoalkenyl aromatic hydrocarbons and conjugated diolefin monomers with glass transition temperatures below 10°C, or d) blocks of conjugated diolefins. These star block polymers can be based on a-c blocks, b-c blocks, a-d blocks or b-d blocks. In certain embodiments of the present disclosure, the number of arms may be 100 or less, or the number of arms may be 50 or less, or the number of arms may be 10 or less, or the number of arms may be 5 or less.

The elastic member 12 may comprise the elastomeric polymer in amounts from about 20% to about 100%, by weight. In other suitable embodiments, the elastic member 12 may comprise the elastomeric polymer in amounts from about 20% to about 80%, or may comprise the elastomeric polymer in amounts from about 30% to about 65%., or may comprise the elastomeric polymer in amounts from about 40% to about 60% Alternatively, the elastic member 12 may comprise the elastomeric polymer in amounts from about 45% to about 60%.

In certain embodiments, elastomeric polymers include styrene-olefin-styrene triblock copolymers such as styrene-ethylene/butylene-styrene (S-EB-S), styrene-ethylene/propylene-styrene (S-EP-S), styrene-ethylene-ethylene/proplyene-styrene (S-EEP-S), and mixtures thereof. The block copolymers may be employed alone or in a blend of block copolymers, and may be partially or fully hydrogenated.

In particular embodiments, the elastomeric polymers include styrene-ethylene/butylene-styrene (S-EB-S), styrene-ethylene/propylene-styrene (S-EP-S), styrene-ethylene-ethylene/proplyene-styrene (S-EEP-S), and hydrogenated styrene-isoprene/vinyl isoprene-styrene. Such linear block copolymers are commercially available under the trade designation Kraton from Kraton Polymers, Houston, TX, and under the trade designations Septon™ and Hybrar™ from Kuraray America, Inc., Pasedena, TX.

The elastic member 12 may comprise one or more modifying resins. Suitable modifying resins may associate or phase mix with the soft blocks of the elastomeric polymer. Modifying resins may have a sufficiently high molecular weight average such that the glass transition temperature of the soft phase is increased resulting in an increase of post elongation strain at 22°C after 15 seconds of recovery. While not intending to be bound by this theory, it is believed that the modifying resins raise the Tg of the soft phase to the point where molecular relaxation at use temperatures is slowed. This is evidenced by a relatively high post elongation strain. In certain embodiments of the present disclosure, the glass transition temperature of the soft phase may be less than about -50°C. In other embodiments of the present disclosure, the glass transition temperature of the soft phase may range from about -50°C to about 35°C. In other embodiments of the present disclosure, the glass transition temperature of the soft phase may range from about - 40°C to about 25°C, while in other embodiments the glass transition temperature of the soft phase may be range from about -30°C to about 20°C. Further, in still other embodiments of the present disclosure, the glass transition temperature of the soft phase may range from about -20°C to about 15°C, while in other embodiments the glass transition temperature of the soft phase may range from about -10°C to about 10°C. The glass transition temperature of the soft phase influences the percent of initial strain after 15 seconds of recovery at 22°C, the level of temperature responsiveness, and the unload force at 37C.

The elastic member 12 may comprise modifying resins in amounts from about 0% to about 60% by weight. In other embodiments, the elastic member 12 may comprise modifying resins in amounts from about 10% to about 55%, or may comprise modifying resins in amounts from about 20% to about 55%, or may comprise modifying resins in amounts from about 30% to about 50%. In certain embodiments, the elastic member 12 may comprise modifying resins in amounts from about 40% to about 50%.

Suitable modifying resins useful herein may have glass transition temperatures ranging from about 60°C to about 180°C, from about 70 °C to about 150 °C, and from about 90 °C to about 130 °C.

Suitable modifying resins may be soft block associating. A solubility parameter is useful in determining whether the modifying resin will phase mix with the soft block of the block copolymer. Generally, modifying resins are selected so that the solubility parameter of the modifying resin is similar to the solubility parameter of the soft block phase. For example in the case where the solubility parameter of the soft block phase is about 8 $(cal/cm^3)^{1/2}$, the solubility parameter of the modifying resin may be from about 7.5 $(cal/cm^3)^{1/2}$ to about 8.5 $(cal/cm^3)^{1/2}$. The solubility parameters of the modifying resins may also approximate the solubility of the hard block. However, so long as the modifying resin phase mixes with the soft block, hard block phase mixing should not be read as limiting. A list of solubility parameters for common polymers or resins, along with methods for determining or approximating the solubility parameters can be found in the Polymer Handbook, Third Edition; Wiley Interscience; Section VII pages 519-559.

Modifying resins useful herein include, but are not limited to, unhydrogenated C5 hydrocarbon resins or C9 hydrocarbon resins, partially and fully hydrogenated C5 hydrocarbon resins or C9 hydrocarbon resins; cycloaliphatic resins; terpene resins; polystyrene and styrene oligomers; poly(t-butylstyrene) or oligomers thereof; rosin and rosin derivatives; coumarone indenes; polycyclopentadiene and oligomers thereof; polymethylstyrene or oligomers thereof; phenolic resins; indene polymers, oligomers and copolymers; acrylate and methacrylate oligomers, polymers, or copolymers; derivatives thereof; and combinations thereof. The resin may be selected from the group consisting of the oligomers, polymers and/or copolymers derived from: t-butylstyrene, cyclopentadiene, iso-bornyl methacrylate, methyl methacrylate, isobutyl methacrylate, indene, coumarone, vinylcyclohexane, methylstyrene, and 3,3,5-trimetbylcyclohexyl methacrylate. Modifying resins may also include alicyclic terpenes, hydrocarbon resins, cycloaliphatic resins, poly-beta-pinene, terpene phenolic resins, and combinations thereof. "C5 hydrocarbon resins" and "C9 hydrocarbon resins" are disclosed in U.S. Patent No. 6,310,154.

The elastic member 12 may comprise a variety of additives. Suitable additives including, for example, stabilizers, antioxidants, and bacteriostats may be employed to prevent Thermal, oxidative, and bio-chemical degradation of the elastic member 12. Additives may account for about 0.01% to about 60% of the total weight of the elastic member 12. In other embodiments, the composition comprises from about 0.01% to about 25%. In other suitable embodiments, the composition comprises from about 0.01% to about 10% by weight, of additives.

Various stabilizers and antioxidants are well known in the art and include high molecular weight hindered phenols (*i.e.,* phenolic compounds with sterically bulky radicals in proximity to the hydroxyl group), multifunctional phenols (*i.e.,* phenolic compounds with sulfur and phosphorous containing groups), phosphates such as tris-(p-nonylphenyl)-phosphite, hindered amines, and combinations thereof. Proprietary commercial stabilizers and/or antioxidants are available under a number of trade names including a variety of Wingstay® Tinuvin® and Irgano® products.

The elastic member 12 may comprise various bacteriostats that are known in the art. Examples of suitable bacteriostats include benzoates, phenols, aldehydes, halogen containing compounds, nitrogen compounds, and metal-containing compounds such as mercurials, zinc compounds and tin compounds. A representative example is available under the trade designation Irgasan PA from Ciba Specialty Chemical Corporation, Tarrytown, NY.

Other optional additives include thermoplastic polymers or thermoplastic polymer compositions which preferentially associate with the hard blocks or segments of the block copolymers. It is possible that these thermoplastic polymers become incorporated into the entangled three-dimensional network structure of the hard phase. This entangled network structure can provide improved tensile, elastic and stress relaxation properties of the elastomeric composition. When the elastomeric polymer comprises a styrenic block copolymer, thermoplastic polymer additives such as polyphenylene oxide and vinylarene polymers derived from monomers including styrene, alpha-methyl styrene, para-methyl styrene, other alkyl styrene derivatives, vinyl toluene, and mixtures thereof, may be useful in embodiments the present disclosure because they are generally considered to be chemically compatible with the styrenic hard blocks of the block copolymer.

The elastic member may comprise equivalent hard block (EHB) polymers. For example, the addition of homopolymer polystyrene for block copolymers comprising substantially polystyrene hard blocks. It is possible that the addition of EHB polymers may provide a reinforcing benefit to the block copolymer composition. Further, it is possible that the degree of interaction between the hard block of the block copolymer and the EHB polymer is dependent on the molecular weight of each species. The ratio of the number average molecular weight of the EHB polymer to the number average molecular weight of the hard block of the block copolymer may range from about 0.5 to about 100, or may range from about 1 to about 100, or may range from about 1 to about 50, or may range from about 1 to about 20. Further, it is possible that the reinforcing benefit may result in increased strength and decreased force relaxation. In certain embodiments of the present disclosure, block copolymer compositions comprising EHB polymers may result in a tensile strength increase of greater than about 5% over the block copolymer composition without the EHB polymer, or greater than about 10%, or greater than about 20%, or greater than about 30%. For thin materials (less than about 1.0 millimeter in thickness), the tensile strength can be determined according to ASTM D 882; while for thick materials (greater that about 1.0 millimeter and less than about 14 millimeters in thickness), the tensile strength can be determined according to ASTM D 638. Further, block copolymer compositions comprising EHB polymers may result in a force relaxation decrease over the block copolymer composition without the EHB polymer. In certain embodiments of the present disclosure, the percentage force loss in tensile mode after 1 hour of relaxation at 37°C and 50% engineering strain of a block copolymer composition comprising a EHB polymer may be less than about 0.99x the block copolymer composition without an EHB polymer, or may be less than about 0.95x, or may be less than about 0.9x, or may be less than about 0.8x, where the percentage force loss can be determined according to ASTM D 6048.

The elastic member 12 may comprise viscosity modifiers, processing aids, slip agents or anti-block agents. Processing aids include processing oils, which are well known in the art and include synthetic and natural oils, naphthenic oils, paraffinic oils, olefin oligomers and low molecular weight polymers, vegetable oils, animal oils, and derivatives of such including hydrogenated versions. Processing oils also may incorporate combinations of such oils. Mineral oil may be used as a processing oil. Viscosity modifiers are also well known in the art. For example, petroleum derived waxes can be used to reduce the viscosity of the slow recovery elastomer in thermal processing. Suitable waxes include low number-average molecular weight (*e.g.,* 0.6-6.0 kilo Daltons) polyethylene; petroleum waxes such as paraffin wax and microcrystalline wax; atactic polypropylene; synthetic waxes made by polymerizing carbon monoxide and hydrogen such as Fischer-Tropsch wax; and polyolefin waxes.

Various colorants and fillers are known in the art and may be included as additives within the composition that forms the elastic member 12. Colorants can include dyes and pigments such as titanium dioxide. Fillers may include such materials as talc and clay. Other additives may include dyes, UV absorbers, odor control agents, perfumes, fillers, desiccants, and the like.

In certain embodiments of the present disclosure, the elastomeric compositions may be microphase separated and it may be desired that the soft phase be substantially continuous while comprising specific anchoring points that include, but are not limited to, one or both of the following: (1) chemical cross-links, and (2) physical cross-links including, but not limited to, one or more of the following-crystalline domains, phase separated blocks, and ionic groups. It is possible that maintaining the soft phase as substantially continuous ensures a relatively high unload force at 37°C and a relatively high post elongation strain at 22°C after 15 seconds of recovery.

For certain embodiments of the present disclosure comprising block copolymer compositions, it may be desirable to have the hard phase form a spherical morphology which may minimize hysteresis. Many commercially available block copolymers suitable for the present disclosure may contain from about 20% by weight to about 40% by weight hard block and may possess cylindrical or lamellar morphologies. To achieve a spherical morphology, it may be advantageous to blend in soft phase associating ingredients that adjust the composition leading to a spherical morphology, *e.g.*, by decreasing the weight percentage of the hard phase in the block copolymer composition. In addition to modifying resins that may raise the soft phase Tg and processing oils that may lower the soft phase Tg, another type of soft phase modifier is an equivalent soft block (ESB) polymer. For example, the addition of poly(ethylene/propylene) for block copolymers comprising an ethylene/propylene soft block such as SEPS, or the addition of a poly(styrene-isoprene) random copolymer of similar repeat unit composition to a polystyrene block copolymer composition comprising a random styrene-isoprene soft block. It is possible that the addition of ESB polymers may provide a hard phase dilution benefit to the block copolymer composition, *e.g.*, leading to a hard phase spherical morphology, and may provide a benefit in maintaining or increasing the order-disorder temperature as compared to other soft block associating ingredients such as modifying resins and processing oils that may reduce the order-disorder temperature. Further, it is possible that the molecular weight of the equivalent soft block polymer may influence the rheology of the ESB polymer and may affect the rheology of the block copolymer composition. In certain embodiments of the present disclosure, the number average molecular weight of an ESB polymer may range from about 0.3 to about 150 kilo Daltons. Further, in certain embodiments of the present disclosure, any combination of soft phase associating ingredients and percentages may be chosen as long as (1) the slow recovery stretch laminate exhibits a percent of initial strain after 15 seconds of recovery at 22°C of about 10% or greater and an unload force at 37°C of about 0.16 N/(g/m) or greater, and comprises a hard phase exhibiting spherical morphology, or (2) the slow recovery elastomer exhibits a percent of initial strain after 15 seconds of recovery at 22°C of about 10% or greater and a normalized unload force at 37°C and 60% hold strain of greater than about 0.07 N, and comprises a hard phase exhibiting spherical morphology.

In certain embodiments of the present disclosure, it has been found that during the use of an absorbent article comprising a stretch laminate comprising an elastic member, certain baby oils, lotions, gels, cremes, and the like, that are spread on the wearer's skin before application of the article, may be absorbed to some extent by the elastic member. It is possible that this behavior may lead to a swelling or breakage of the elastic member and may result in reduced performance. Swelling may lead to (1) a sticky feeling slow recovery stretch laminate that may cause discomfort to the wearer of the absorbent article, and/or (2) reduction in the unload force of the slow recovery stretch laminate at 37C, which may result in poor fit and may lead to, for example, increased urine or bowel movement leakage during use, sagging or drooping of the absorbent article during use, and/or increased discomfort in wearing the absorbent article. Breakage of the elastic member may lead to poor fit if it is localized, but if widespread may lead to catastrophic failure of the slow recovery stretch laminate which may lead to the failure of the absorbent article comprising the slow recovery stretch laminate.

It is possible that for stretch laminates comprising an elastic member comprising a block copolymer composition that certain ingredients of baby oils, lotions, gels, cremes, and the like, may weaken or solubilize the hard block or hard phase domains of the elastic member. In certain embodiments of the present disclosure, this behavior is believed to lead to a dissolution of physical cross-links (hard phases) and thereby a weakening or breakage of the elastic member. The solubility parameter of the hard block is useful for determining whether the hard block may be susceptible to solubilization by ingredients of baby oils, lotions, gels, cremes, and the like. In certain embodiments of the present disclosure, the solubility parameter of the hard block may be greater than 9.1 $(cal/cm^3)^{1/2}$, or may be greater than 9.3 $(cal/cm^3)^{1/2}$, or may be greater than 9.5 $(cal/cm^3)^{1/2}$, where the solubility parameter of the hard block is determined according to the method described by L.H. Sperling in Introduction to Physical Polymer Science, Wiley-Interscience (New York, 1992). For example, according to the method described by Sperling, the solubility parameter for polystyrene hard blocks is determined to be 8.96 $(cal/cm^3)^{1/2}$. Further, in certain embodiments of the present disclosure, it has been found that exposure of an elastic member comprising substantially polystyrene hard blocks to baby oils containing ingredients like, but not limited to, isopropyl palmitate may lead to disintegration of the elastic member wherein the elastic member becomes fragile and may deteriorate into stringy-like pieces. According to the method described by Sperling, the solubility parameter of isopropyl palmitate is determined to be 8.12 $(cal/cm^3)^{1/2}$, indicating that negative effects may occur to an elastic member exposed to ingredients like isopropyl palmitate when the solubility parameter differences between the hard block and such ingredients is about 0.84 $(cal/cm^3)^{1/2}$ or less. Further, in certain embodiments of the present disclosure, it has been found that elastic members which survive exposure to mineral oil and ispropyl palmitate, as described in the Oil Exposure Method, may be considered resistant to baby oils, lotions, gels, cremes, and the like, when spread on the wearer's skin before application of an absorbent article comprising a slow recovery stretch laminate comprising the elastic member. Still further, in contrast to the high glass transition temperature hard block modifiers described above, ingredients of baby oils, lotions, gels, cremes, and the like, that may cause a negative impact on elastic members may have a glass transition temperature well below room temperature, e.g., may be liquid at room and use temperatures, and it is possible that exposure of the elastic member to such ingredients may result in a hard phase glass transition temperature below use temperature and may result in a weakening and possible dissolution of the elastic member. Additionally, it is possible that ingredients of baby oils, lotions, gels, cremes, and the like, which are liquid at use temperature may require a larger difference between the solubility parameter of such ingredients and the solubility parameter of the hard block in order for the hard block to remain intact after exposure to such ingredients as compared to the difference between the solubility parameter of modifying resins, which are typically solid at use temperature, and the solubility parameter of the soft block in order for the modifying resin to be soft block associating, as described above.

For stretch laminates comprising an elastic member comprising a block copolymer composition comprising a hard or soft block comprising polystyrene, possible means of increasing the resistance of the elastic member to certain ingredients of baby oils, lotions, gels, cremes, and the like include aromatic substitution with nitro, chlorine, bromine, or nitrile groups, ketone groups of various structures including methyl, ethyl, propyl, and butyl ketones, ester groups of various structures including methyl, ethyl, propyl, butyl, pentyl and hexyl esters, mono- and di-substituted amide groups of various structures including methyl, ethyl, propyl, butyl, phenyl, and benzyl amides, and combinations thereof. For block copolymers comprising a hard or soft block comprising polystyrene, the degree of substitution is greater than 10% and may be less than 300%, or the degree of substitution may be less than 200%, or the degree of substitution may be less than 100%, or the degree of substitution may be greater than 20% and less than 70%, or the degree of substitution may be greater than 30% and less than 60%, where a degree of substitution of 50% refers to an average of 1 substituted group per two aromatic rings of the block copolymer, a degree of substition of 100% refers to an average of 1 substituted group per aromatic ring, a degree of substition of 200% refers to an average of 2 substituted groups per aromatic ring, and a degree of substitution of 300% refers to an average of 3 substituted groups per aromatic ring. Further, to minimize chain scission within the soft block during aromatic substitution, the degree of hydrogenation of the soft block backbone may be greater than about 70 mole percent of the soft block backbone, or the degree of hydrogenation may be greater than about 80 mole percent of the soft block backbone, or the degree of hydrogenation may be greater than about 90 mole percent of the soft block backbone, or the degree of hydrogenation may be greater than about 95 mole percent of the soft block backbone, or the degree of hydrogenation may be greater than about 99 mole percent of the soft block backbone.

Further, it is possible to increase the resistance of the elastic member to certain ingredients of baby oils, lotions, gels, cremes, and the like, through the use of polymer skin layers on the elastic member, *e.g.,* an A-B-A (3-layer) film construction where the A layers represent the polymer skins and the B layer an elastomer, *e.g.*, a slow recovery elastomer. For certain embodiments of the present disclosure, the polymer skin layers may comprise thermoplastic elastomers based on, but not limited to, polyurethanes, polyesters, polyether amides, elastomeric polyolefins including polyethylenes and polypropylenes, elastomeric polyolefin blends, and combinations thereof. In other embodiments of the present disclosure, the polymer skin layers may comprise thermoplastic polymers based on, but not limited to, polyolefin polymers including polyethylenes and polypropylenes, and combinations thereof. Further, for certain embodiments of the present disclosure, the polymer skin layers may comprise combinations of thermoplastic elastomers and thermoplastic polymers. Additionally, other multilayer film constructions are also within the scope of the present disclosure including, but not limited to, A-B-C, A-B-A-B-A, and A-B-C-B-A type constructions. Further, the use of polymer skins may also provide an antiblock benefit, reducing the tendency of the elastic member to stick to itself, to substrates, or to processing equipment.

Further, it is possible to increase the resistance of the elastic member to certain ingredients of baby oils, lotions, gels, cremes, and the like, through the use of particulate materials incorporated onto the outer surfaces of the elastic member. For certain embodiments of the present disclosure, suitable particulate materials include, but are not limited to, inorganic minerals such as talc, calcium carbonate, clays, titanium dioxide, tricalcium phosphate, and silica, *e.g.*, Aerosil® 90 available from Evonik Degussa Corporation, Piscataway, NJ, organic particulates such as starch and cellulose, and combinations thereof. Further, the use of particulate materials may also provide an antiblock benefit, reducing the tendency of the elastic member to stick to itself, to substrates, or to processing equipment.

Further, it is possible to increase the resistance of the elastic member to certain ingredients of baby oils, lotions, gels, cremes, and the like, through the use of antiblock agents applied in a fluid or molten state to the outer surfaces of the elastic member. For certain embodiments of the present disclosure, suitable antiblock agents and suitable processes for applying the antiblock agents are disclosed in U.S. Application Nos. 11/413,483 and 11/413,545 filed on April 28, 2006 in the name of Arman Ashraf and Daniel Steven Wheeler which claims the benefit of U.S. Provisional Application Nos. 60/676,755 and 60/676,275, respectively, filed on April 29, 2005.

Suitable substrates 14 (shown in Figs. 1A-E) for use in a stretch laminate 10 include nonwoven webs, woven webs, knitted fabrics, films, film laminates, apertured films, nonwoven laminates, sponges, foams, scrims, and any combinations thereof. Suitable substrates may comprise natural materials, synthetic materials, or any combination thereof. For use in absorbent articles and particularly in diapers and like products, the substrate 14 is generally compliant, soft-feeling, and non-irritating to a wearer's skin. In certain embodiments, substrates 14 may include nonwoven webs such as spunbond webs, meltblown webs, carded webs, and combinations thereof (*e.g.*, spunbond-meltblown composites and variants).

The dimensions of the substrate 14 are generally limited only by the requisite end-use of the stretch laminate 10.

The stretch laminate 10 embodiments of the present disclosure may exhibit unique elastic and recovery characteristics. The stretch laminate 10 may exhibit a normalized unload force of greater than about 0.16 N/(g/m) at 37°C as measured by the Two Cycle Hysteresis Test. Normalized unload forces of less than about 0.12 N/(g/m) at 37 °C may not be sufficient for use as an elastomer within absorbent articles. Laminates having normalized unload forces less than 0.12 N/(g/m) at 37°C may be unable to keep an absorbent article in snug, close contact to the wearer's skin. In certain embodiments, the stretch laminate 10 may exhibit a normalized unload force of greater than about 0.24 N/(g/m) at 37°C, or may exhibit a normalized unload force of greater than about 0.36 N/(g/m) at 37°C, or may exhibit a normalized unload force of greater than about 0.48 N/(g/m) at 37°C, or may exhibit a normalized unload force of greater than about 0.60 N/(g/m) at 37°C.

Conventional stretch laminates (*i.e.,* such as those commonly found in absorbent articles including diapers) may exhibit minimal post elongation strain at 22°C after 15 seconds of recovery. Qualitatively, conventional stretch laminates exhibit "snap back" (*i.e.,* contracts relatively quickly after being released from a stretched state). In contrast, a slow recovery stretch laminate (SRSL) 10 of the current invention may exhibit a percent of initial strain of about 10% or greater after 15 seconds of recovery at 22°C, as measured by the Post Elongation Recovery Test. In other embodiments, an SRSL 10 may exhibit a percent of initial strain of about 20% or greater after 15 seconds of recovery at 22°C. In other suitable embodiments, an SRSL 10 may exhibit a percent of initial strain of about 30% or greater after 15 seconds of recovery at 22°C. In other suitable embodiments, an SRSL 10 may exhibit a percent of initial strain of about 40% or greater after 15 seconds of recovery at 22°C.

Furthermore, an SRSL 10 embodiment of the present disclosure may exhibit a specified percent of initial strain at 22°C after 30 seconds, 60 seconds, or three minutes of recovery. In certain embodiments, an SRSL 10 may exhibit a percent of initial strain at 22°C after 30 seconds of recovery of about 10% or greater. In other embodiments, an SRSL 10 may exhibit a percent of initial strain at 22°C after 30 seconds of recovery about 15% or greater. In other embodiments, an SRSL 10 may exhibit a percent of initial strain at 22°C after 60 seconds of recovery of about 10% or greater.

An SRSL 10 may exhibit temperature responsiveness. In certain embodiments, an SRSL 10 may exhibit a percent of initial strain at 37°C after a specified amount of recovery time that is less than the percent of initial strain exhibited at 22°C after the same recovery time. In one embodiment, a temperature responsive SRSL 10 may exhibit a reduction in a percent of initial strain after 15 seconds at 37°C as compared to the percent of initial strain exhibited after 15 seconds at 22°C (*i.e.,* [percent of initial strain after 15 seconds of recovery at 22°C]-[percent of initial strain after 15 seconds of recovery at 37°C]). In some embodiments, the difference is equal to or greater than 5%. In other embodiments, an SRSL 10 may exhibit a difference in the percent of initial strain after 15 seconds at 22°C compared to after 15 seconds at 37 °C equal to or greater than 10%, 20%, 30%, or 40%. It is believed that an SRSL 10 exhibiting temperature responsiveness may further facilitate diaper application. When the diaper is applied at about room temperature (*i.e.,* approximately 22°C), an SRSL 10 may exhibit a relatively high percent of initial strain for a prescribed period of time, which allows the caregiver or wearer to apply the diaper. Upon application of the diaper, the temperature of an SRSL 10 will rise as a result of being in close proximity to the wearer's skin. As the temperature of an SRSL 10 increases and nears body temperature (*i.e.,* approximately 37°C), the percent of initial strain is reduced. Temperature responsiveness allows for application of the diaper without "snap-back" while providing for increased recovery after application.

An SRSL 10 may be utilized in a variety of consumer and commercial products. However, an SRSL 10 has particular benefit within absorbent articles, particularly disposable absorbent articles such as diapers and the like. An SRSL 10 may be used in a variety of regions or in a variety of article elements to provide elastic character to the absorbent article. It may be desirable to incorporate an SRSL 10 embodiments of the present disclosure into the absorbent articles disclosed in U.S. Pub. Nos. 2005-0273071, 2005-0171499, 2007-0191806, 2004-0162538, and 2005-0095942.

Another embodiment of the present disclosure is directed toward a method of applying any of the absorbent articles as disclosed above. The absorbent article may be provided to a caregiver for application onto a wearer. The absorbent article may be in a compacted state such that a stretch laminate comprising an SRSL is in a relaxed, substantially untensioned state. The caregiver may stretch the absorbent article thereby expanding and tensioning the stretch laminate. The article is generally stretched in preparation for application. The absorbent article can maintain a functionally elongated state for an effective period of time. In one embodiment, the article may maintain an elongated state for a sufficient amount of time necessary for the caregiver to apply the article to the wearer. With conventional diapers (not comprising SRSL), upon release of the diaper after stretching, the diaper often contracts and/or folds before it can be successfully applied to a wearer. In one embodiment, SRSL may exhibit a percent of initial strain after 15 seconds of recovery at 22°C of greater than or equal to 10%. After application, the article may continue to contract so as to provide a snug, ideal fit.

In another embodiment, a plurality of absorbent articles as disclosed above may be packaged in a kit. Generally, the kit allows for a quantity of absorbent articles to be delivered to and purchased by a consumer while economizing space and simplifying transport and storage. The kit may require activation so that the article becomes accessible (*e.g.*, opening of a lid, removal of a panel, etc.). In one embodiment, the kit is defined by numerous absorbent articles bound together as an entity and covered by a thermoplastic film overwrap as disclosed in U.S. Patent No. 5,934,470. The thermoplastic film cover may contain an opening means to allow removal of a portion of the thermoplastic film cover and access to the articles. An opening means may include a substantially continuous line of weakness, including perforations within the thermoplastic film cover. An opening means that may be used is presented in U.S. Pat. App. No. 5,036,978.

While one kit embodiment is described above, other variations to the kit are clearly envisioned. The overwrap may comprise a variety of materials including, but not limited to, thermoplastic films, nonwovens, wovens, foils, fabrics, papers, cardboard, elastics, cords, straps, and combinations thereof. The overwrap may completely or partially bind and/or cover the plurality of absorbent articles. Other useful packages and methods for packaging are disclosed in U.S. Patent Nos. 5,050,742 and 5,054,619. Furthermore, a kit may contain multiple overwraps. For example, a plurality of absorbent articles of the present disclosures may be packaged with a thermoplastic film overwrap and then a plurality of film wrapped pull-on garments being overwrapped in a cardboard box or a second thermoplastic film overwrap. Furthermore, the kit may not contain a dedicated opening means. For example, a thermoplastic film overwrap without perforation may simply be opened by tearing the film.

TEST METHODS

POST ELONGATION RECOVERY

This method is used to determine the post elongation strain of a stretch laminate as a function of temperature and time, and with certain variations as described below is used to determine the post elongation strain of an elastomer as a function of temperature and time. The measurement is done at 22 °C (72°F) or at 37°C (99°F). The measurement at 22°C (72°F is designed to simulate the recovery of the stretch laminate (or elastomer) at room temperature, while the measurement at 37°C (99°F) is designed to measure the recovery of the stretch laminate (or elastomer) near body temperature. Other test temperatures are within the scope of this method including, but not limited to, the recovery of the stretch laminate (or elastomer) near skin temperature (32°C). A two-step analysis, Stretch and Recovery, is performed on the samples. The method employs a Dynamic Mechanical Analyzer. A TA Instruments DMA Q800 (hereinafter"DMA Q800'), available from TA Instruments, Inc., of New Castle, Delaware; equipped with a film clamp, Thermal Advantage/Thermal Solutions software for data acquisition, and Universal Analysis 2000 software for data analysis was used herein. Many other types of DMA devices exist, and the use of dynamic mechanical analysis is well known to those skilled in the art of polymer and copolymer characterization.

Methods of operation, calibration and guidelines for using the DMA Q800 are found in TA Instruments DMA Q800 Getting Started Guide issued July 2007, Thermal Advantage Q Series™ Getting Started Guide issued February 2004 and Universal Analysis 2000 guide issued May 2004. To those skilled in the use of the DMA Q800, the following operational run conditions should be sufficient to replicate the stretch and recovery of the samples.

The DMA Q800 was configured to operate in the Controlled Force Mode with the film clamp. The film clamp is mounted onto the DMA Q800 and calibrated according to the User's Reference Guide. The stretch laminate (or elastomer) to be tested is cut into samples of substantially uniform dimension. For the DMA Q800, suitable sample dimensions are approximately 20 mm x 6.4 mm x 1.0 mm (length x width x thickness). The sample thickness of the stretch laminate is dependent on the materials and structure of the stretch laminate and on the confining pressure used to measure the thickness. TA Instruments recommends the sample thickness, when securely mounted within the film clamps, to be less than or equal to about 2.0 mm. The lower film clamp of the DMA Q800 is adjusted and locked in a position which provides approximately 10 mm between the clamping surfaces. The sample is mounted in the film clamps and the lower clamp is allowed to float to determine the gauge length between the film clamps. It should be understood that the sample referenced in this method is one where the SRSL must run from the upper clamp to the lower clamp. The sample ID and dimensions are recorded. The film clamp is locked in position and the furnace is closed.

Stretch Method-For the sample dimensions specified above, the DMA Q800 is configured as follows: Preload force applied to sample in clamp (0.01N); auto zero displacement (on) at the start of the test; furnace (close), clamp position (lock), and temperature held at $T_i$ (22°C or 37°C) at the end of the stretch method. Data acquisition rate is set at 0.5 Hz (1 point per 2 seconds). The stretch method is loaded onto the DMA Q800. The method segments are (1) Initial Temperature $T_i$ (22°C or 37°C), (2) Equilibrate at $T_i$ (3) Data Storage ON, and (4) Ramp Force 5.0 N/min to 18.0 N.

Upon initiation of the test, the temperature ramps to the specified $T_i$ (22°C or 37°C) [method segment 1], and the temperature is maintained at this $T_i$ [method segment 2]. After a minimum of 15 minutes at $T_i$, the operator initiates the sample stretching and concurrent data collection [method segments 3 and 4]. The sample is stretched with an applied ramp force of 0.8 N/min per millimeter of initial sample width (*e.g.*, for the sample dimensions specified above, the applied ramp force is 5 N/minute) to approximately 30 mm in length. The gradual increase in force more closely simulates application of the article and prevents sample breakage. The sample is locked in place at the stretched length of approximately 30 mm and maintained at $T_i$. The force required to stretch the laminate to a length of approximately 30 mm and the percent strain of the laminate at this length are recorded manually from the digital readout on the instrument. The percent strain is calculated by subtracting the gauge length from the stretched length, then dividing the result by the gauge length and multiplying by 100. The initial percent strain is described by the equation below:

$$\text{Initial Percent Strain} = \%\text{Strain}_i = 100*[(L_s - L_g)/L_g].$$

where $L_g$ is the gathered length of the relaxed stretch laminate (or elastomer) between the film clamps at the beginning of the stretch step, and $L_s$ is the length of the stretched laminate (or elastomer) between the film clamps at the end of the stretch step of the analysis (~30 mm). The $\%\text{Strain}_i$ is the percent strain of the stretch laminate (or elastomer) at the start of the recovery method (*i.e.,* after the stretch part of the method is complete). A sample stretched from a gauge length of 10 mm to a length of 30 mm results in a percent strain of 200%.

Stretch laminates may be unable to exhibit extensibility of 200% strain without incurring irreversible deformation, delamination, tearing, or a significant percent set (*i.e.,* set of greater than about 10%). This is particularly true for stretch laminates obtained from commercially available products such as the side panels, leg cuffs and waistbands of diapers. For example, a stretch laminate (~6.4 mm wide) may be easily stretched to 100% strain or 150% strain when relatively low forces (< 4N) are applied. However, if the applied force continues to increase to achieve 200% strain, the percent strain of the stretch laminate plateaus and further extension may be difficult and/or may result in irreversible deformation, delamination, tearing, or significant percent set (*i.e.,* set of greater than 5%) of the stretch laminate. For stretch laminates, the initial percent strain ($\%\text{Strain}_i$) is taken as 70% of the average maximum percent strain determined according to the Maximum Laminate Strain Test rounded up to the nearest multiple of five if the value does not result in a target strain that is divisible by five when rounded to the nearest percent. For example, if 70% of the average maximum percent strain is equal to 187.1%, this value is not divisible by 5 when rounded to the nearest percent (187%), so the initial percent strain would be taken as 190%. Also, for example, if 70% of the average maximum percent strain is equal to 180.1%, this value is divisible by 5 when rounded to the nearest percent (180%), so the peak strain would be taken as 180%. For laminates with an average maximum percent strain of greater than 536%, an initial percent strain of 375% is used. For elastomers or elastomeric films (or stretch films), the initial percent strain is 400%. The required gathered length of the relaxed laminate (or elastomer) between the film clamps at the beginning of the stretch step can be approximated from the initial percent strain determined above using the initial percent strain equation above.

For samples of different dimensions, the applied force to stretch the sample is adjusted to achieve an applied ramp force of 0.8 N/min per millimeter of initial sample width. For example, a force ramp of 2.5 N/min is applied to a sample with an initial width of 3.2 mm.

Recovery Method-The Recovery Method is loaded onto the instrument and initiated approximately 15 seconds after reaching the desired initial percent strain ($\%\text{Strain}_i$) in the Stretch Method. The four segments of the recovery method are (1) Data Storage ON, (2) Force 0.01N, (3) Ramp to $T_i$, and (4) Isotherm for 3.0 minutes. The following DMA Q800 parameter setting is changed from the Stretch Method: auto zero displacement is changed to (OFF). The Recovery Method measures the length of the sample over a 3 minute time period at the specified temperature ($T_i$ = either 22°C or 37°C). The sample length, percent strain, and test temperature are recorded as a function of recovery time. The post elongation strain is reported as the percent of the initial percent strain after different times of recovery (15 seconds, 30 seconds, 60 seconds, and 3 minutes). For example, if the initial percent strain is 400% and the percent strain after 15 seconds of recovery at 22°C is 50%, then the percent of initial strain after 15 seconds of recovery at 22°C is reported as 12.5% (= 100 x 50%/400%).

For samples of different dimensions, the force applied to the sample during recovery (segment 2 above) is adjusted to achieve an applied force of 0.0016 N per millimeter of initial sample width (0.01N for 6.4 mm wide sample). For example, a force of 0.005 N is applied to a sample 3.2 mm wide.

MAXIMUM LAMINATE STRAIN

The maximum laminate strain is that strain above which a stretch laminate may sustain irreversible deformation, delamination, tearing, or a significant percent set (*i.e.,* set of greater than 5%). In a tensile test, such as described in ASTM D 882, the maximum laminate strain may be associated with a relatively steep rise in the tensile force with increasing tensile strain (not including the initial rise that occurs at very low strains, often at less than about 15 percent engineering strain).

The tensile test is performed at room temperature (about 22°C). The stretch laminate to be tested is cut into a sample of substantially rectilinear dimensions. Sample dimensions are selected to achieve the required strain with forces appropriate for the instrument. Suitable instruments for this test include tensile testers from MTS Systems Corp., Eden Prairie, Minn. (*e.g.* Alliance RT/1 or Sintech 1/S) or from Instron Engineering Corp., Canton, Mass. For either the Alliance RT/1 or Sintech 1/S instruments listed above, suitable sample dimensions are approximately 16 millimeters wide by approximately 75 millimeters long.

The following procedure illustrates the measurement when using the above sample dimensions and either an Alliance RT/1 or Sintech 1/S. The instrument is interfaced with a computer. TestWorks 4™ software controls the testing parameters, performs data acquisition and calculation, and provides graphs and data reports.

The width of the grips used for the test is greater than or equal to the width of the sample. 1 inch (2.54 cm) wide grips may be used. The grips are air actuated grips designed to concentrate the entire gripping force along a single line perpendicular to the direction of testing stress having one flat surface and an opposing face from which protrudes a half round (radius = 6 mm) to minimize slippage of the sample.

The load cell is selected so that the forces measured will be between 10% and 90% of the capacity of the load cell or the load range used. A 25 Newton load cell may be appropriate. The fixtures and grips are installed. The instrument is calibrated according to the manufacturer's instructions. The distance between the lines of gripping force (gage length) is 1.0 inch (25.4 millimeters), which is measured with a steel ruler held beside the grips. The load reading on the instrument is zeroed to account for the mass of the fixture and grips. The specimen is equilibrated a minimum of 1 hour at about 22°C before testing. The specimen is mounted into the grips in a manner such that there is no slack and the load measured is between 0.00 Newton's and 0.02 Newton's. The instrument is located in a temperature-controlled room for measurements performed at about 22°C and a crosshead speed of 20 inches (50,8cm) per minute. The test is initiated and the specimen is extended at 20 in/min (50,8cm/min) until it breaks. The data acquisition rate is 200 Hertz for strains up to about 40% engineering strain, and 50 Hertz for strains above about 40%.

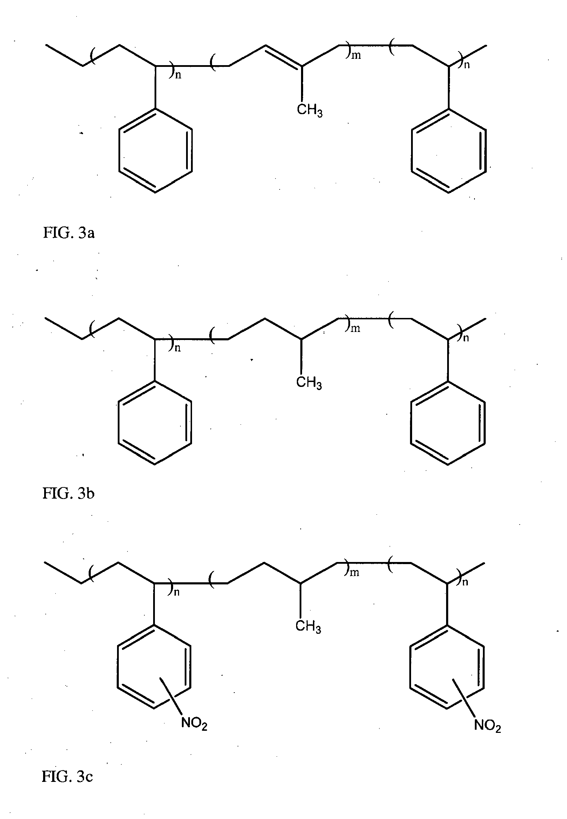

Outputs of the tensile test include the sample strain (engineering strain) and the corresponding tensile force, where an example of an inventive material (Laminate Sample L2, Table 5) is shown in Fig. 3. There may be a small rise **120** in the force at low strains, followed by a relatively flat plateau region **121** corresponding to the primary stretch region of the laminate, and then past the stretch region there is a steep rise **122** in the measured force with increasing sample strain. The maximum laminate strain is taken as the intersection **123** of two linear lines drawn on the tensile force-strain curve: (i) the plateau region **124,** and (ii) the steep rise region **125.** For samples that exhibit a yield drop (such as shown in Fig. 3), line **124** is the tangent line to the force-strain curve at the highest strain value **128** between the initial rise **120** and steep rise **122** regions where the slope of the force-strain curve is equal to zero. For samples that don't exhibit a yield drop, line **124** is the tangent line to the force-strain curve at the strain value between the initial rise **120** and steep rise **122** regions where the slope of the force-strain curve reaches its minimum value. Line **125** is the tangent line at strain point **127** where the slope of the force-strain curve reaches its first peak value in the steep rise region **122,** *e.g.,* where the second derivative of the force-strain curve is equal to zero. For the crosshead speed and data acquisition rates mentioned above, the tangent line at each strain point is taken as the slope of a linear regression line through the following seven force-strain data points-(i) the force-strain value at the specific strain, (ii) the force-strain values at the three strain points before the specific strain, and (iii) the force-strain values at the three strain points after the specific strain. The number of data points per regression line are chosen such that there is less than a 5% change in the strain value at the intersection **123** upon increasing and decreasing the number of data points per regression line in increments of two, *e.g.*, increasing the number of data points from seven points per regression line to nine points per regression line, where in all cases the regression is set up so there is an equal number of data points before and after the specific strain included in the linear regression analysis. A minimum of five samples of each stretch laminate is measured for its maximum laminate strain, and the arithmetic average is reported as the maximum laminate strain.

TWO CYCLE HYSTERESIS TEST FOR LAMINATES

This method is used to determine laminate properties that may correlate with the forces experienced by the consumer during application of the product containing a slow recovery stretch laminate and how the product fits and performs once it is applied.

The two cycle hysteresis test method is performed at room temperature (about 22°C) and also at body temperature (37C). The stretch laminate to be tested is cut into a sample of substantially rectilinear dimensions. Sample dimensions are selected to achieve the required strain with forces appropriate for the instrument. Suitable instruments for this test include tensile testers from MTS Systems Corp., Eden Prairie, Minn. (*e.g.* Alliance RT/1 or Sintech 1/S) or from Instron Engineering Corp., Canton, Mass. For either the Alliance RT/1 or Sintech 1/S instruments listed above, suitable sample dimensions are approximately 16 millimeters wide by approximately 75 millimeters long. The sample thickness is dependent on the materials and structure of the stretch laminate and on the confining pressure used to measure the thickness. The thicknesses of samples may be 0.5 millimeters to 5 millimeters thick measured with 0.2 pounds per square inch ($35,72g/cm^2$) confining pressure. However, testing of stretch laminates with different thicknesses (*e.g.*, less than 0.5 millimeters or greater than 5 millimeters) is within the scope of this method.

The following procedure illustrates the measurement when using the above sample dimensions and either an Alliance RT/1 or Sintech 1/S. The instrument is interfaced with a computer. TestWorks 4™ software controls the testing parameters, performs data acquisition and calculation, and provides graphs and data reports.

The width of the grips used for the test is greater than or equal to the width of the sample. 1 inch (25.4 millimeter) wide grips may be used. The grips are air actuated grips designed to concentrate the entire gripping force along a single line perpendicular to the direction of testing stress having one flat surface and an opposing face from which protrudes a half round (radius = 6 mm) to minimize slippage of the sample.

The load cell is selected so that the forces measured will be between 10% and 90% of the capacity of the load cell or the load range used. A 25 Newton load cell may be used. The fixtures and grips are installed. The instrument is calibrated according to the manufacturer's instructions. The distance between the lines of gripping force (gauge length) is 2.50 inches (63.5 millimeters), which is measured with a steel ruler held beside the grips, unless specified otherwise. The load reading on the instrument is zeroed to account for the mass of the fixture and grips. The specimen is equilibrated a minimum of 1 hour at 22°C before testing. The specimen is mounted into the grips in a manner such that there is no slack and the load measured is between 0.00 Newton's and 0.02 Newton's, unless specified otherwise. The instrument is located in a temperature-controlled room for measurements performed at 22°C. A suitable environmental chamber is used to maintain the testing temperature for measurements performed at 37°C; the sample is mounted in the grips and equilibrated for 5 minutes at 37C before starting the test.

Stretch laminates from different sources may have different strains above which irreversible deformation, delamination, tearing, or a significant percent set (*i.e.,* set greater than 5%) begins to occur. This is true for stretch laminates obtained from commercially available products such as the side panels, leg cuffs, and waistbands of diapers, and for stretch laminates made internally, for example stretch laminates made according to the Laminate Preparation Method disclosed in the Examples. For the purposes of the Two Cycle Hysteresis Test for Laminate Samples, the peak test strain ($\%Strain_{peak}$) is taken as 70% of the average maximum percent strain determined according to the Maximum Laminate Strain Test rounded up to the nearest multiple of five if the value does not result in a target strain that is divisible by five when rounded to the nearest percent. For example, if 70% of the average maximum percent strain is equal to 187.1%, this value is not divisible by 5 when rounded to the nearest percent (187%), so the peak strain would be taken as 190%. Also, for example, if 70% of the average maximum percent strain is equal to 180.1%, this value is divisible by 5 when rounded to the nearest percent (180%), so the peak strain would be taken as 180%. For laminates with an average maximum percent strain of greater than 536%, a peak strain of 375% is used.

The two cycle hysteresis test method for laminate samples involves the following steps (all strains are engineering strains):

(1) Strain the sample to the specified peak percent strain (%Strain peak) at a constant crosshead speed of 20 inches per minute (50.8 centimeters per minute) with no hold.
(2) Reduce the strain to 0% strain (*i.e.,* return grips to the original gage length of 2.50 inches) at a constant crosshead speed of 3 inches per minute (7.62 centimeters per minute) with no hold.
(3) Strain the sample to $\%Strain_{peak}$ at a constant crosshead speed of 20 inches per minute (50.8 centimeters per minute) with no hold.
(4) Reduce the strain at a constant crosshead speed of 3 inches per minute (7.62 centimeters per minute) to the first hold strain listed in Table 1 for the specified %Strain$_{peak}$.

(5) Hold the sample at the strain in step (4) for the first hold time listed in Table 1 for the specified %Strain$_{peak}$.

(6) Reduce the strain at a constant crosshead speed of 3 inches per minute (7.62 centimeters per minute) to the second hold strain listed in Table 1 for the specified %Strain$_{peak}$.

(7) Hold the sample at the strain in step (6) for the second hold time listed in Table 1 for the specified %Strain$_{peak}$.

(8) Reduce the strain at a constant crosshead speed of 3 inches per minute (7.62 centimeters per minute) to the third hold strain listed in Table 1 for the specified %Strain$_{peak}$.

(9) Hold the sample at the strain in step (8) for the third hold time listed in Table 1 for the specified %Strain$_{peak}$.

(10) Reduce the strain at a constant crosshead speed of 3 inches per minute (7.62 centimeters per minute) to the fourth hold strain listed in Table 1 for the specified %Strain$_{peak}$.

(11) Hold the sample at the strain in step (10) for the fourth hold time listed in Table 1 for the specified %Strain$_{peak}$.

(12) Go to 0% strain at a constant crosshead speed of 3 inches per minute (7.62 centimeters per minute).

For laminates in which the test %Strain$_{peak}$ is greater than or equal to 60%, and where one of the hold strains in steps (5), (7), (9), or (11) is equal to 60%, the reported unload force is the measured unload force of the stretch laminate (SL) at 60% after the hold period, normalized to Newton's per meter width of SL per grams per square meter basis weight of elastomer plus adhesive (E+A) in the SL, N/(m·gsm) = N/(g/m), as shown in the equation below. The basis weight of the elastic and adhesive in the SL is calculated by dividing the grams of elastomer plus adhesive in the SL by the area of the SL fully extended. The area of the fully extended stretch laminate (A$_{FESL}$) is defined as the area of the substrate of the stretch laminate in the absence of elastic and adhesive. The normalized unload force in N/(m·gsm) = N/(g/m) =

$$\frac{\text{measured unload force in Newtons}}{\left\{ \left[ \text{width of SL in meters} \right] \times \left[ \left( \text{grams of E+A} \right) \div \left( A_{FESL} \text{ in square meters} \right) \right] \right\}} .$$

A minimum of five samples for each material is tested, and the arithmetic average of the normalized unload force at 60% strain is reported.

For laminates in which the test %Strain$_{peak}$ is greater than or equal to 60%, and where none of the hold strains in steps (5), (7), (9), or (11) is equal to 60%, the reported unload force is the interpolated unload force of the stretch laminate at 60% strain obtained by interpolating linearly between the unload forces at adjacent strains and then normalizing according to the equation above. For example, if %Strain$_{peak}$ = 180%, then according to Table 1, the hold strains for steps (5), (7), (9), and (11) are 100%, 75%, 50%, and 30%, respectively, and the unload force at 60% would be determined by interpolating linearly between the unload forces obtained in steps (7) and (9) after the hold period, using for example the following relationship:

$$UL_{60} = UL_{60+} - \left( UL_{60+} - UL_{60-} \right) \left[ \frac{HS_{60+} - 60\%}{HS_{60+} - HS_{60-}} \right]$$

Where $UL_{60}$ is the unload force at 60% strain after the hold period, $UL_{60+}$ and $UL_{60-}$ are the unload forces at hold strains just above and just below 60% strain after the hold period, respectively, and $HS_{60+}$ and $HS_{60-}$ are the hold strains just above and just below 60% strain, respectively. Therefore, for the example above, if the unload force in step (7) at 75% strain after the hold is 1.04 Newton's and the unload force in step (9) at 50% strain after the hold is 0.78 Newton's, the interpolated unload force at 60% strain after the hold period is 0.88 Newton's:

$$UL_{60} = 1.04N - \left( 1.04N - 0.78N \right) \left[ \frac{75\% - 60\%}{75\% - 50\%} \right] = 0.88N$$

The interpolated unload force is then normalized according to the equation above. A minimum of five samples for each material is tested, and the arithmetic average of the normalized interpolated unload force at 60% strain is reported.

For laminates in which the test %Strain$_{peak}$ is less than 60%, the reported unload force is the measured unload force of the stretch laminate in step (5) after the hold period, normalized according to the equation above. A minimum of five samples for each material is tested, and the arithmetic average of the normalized unload force from step (5) after the hold period is reported. For different sample dimensions, the crosshead speed is adjusted to maintain the appropriate strain rate for each portion of the test. For example, for a sample gauge length of 1.25 inches (31.7 millimeters), a crosshead speed of 10 inches per minute (25.4 centimeters per minute) would be used in Steps 1 and 3, and a crosshead speed of 1.5 inches per minute (3.81 centimeters per minute) would be used in Steps 4, 6, 8, 10 and 12.

TABLE 1. Hold Strains and Times for Two Cycle Hysteresis Test for Laminate Samples

| Peak Percent Strain | First Hold Percent Strain (Steps 4 and 5) | First Hold Time (Minutes) (Step 5) | Second Hold Percent Strain (Steps 6 and 7) | Second Hold Time (Minutes) (Step 7) | Third Hold Percent Strain (Steps 8 and 9) | Third Hold Time (Minutes) (Step 9) | Fourth Hold Percent Strain (Steps 10 and 11) | Fourth Hold Time (Minutes) (Step 11) |
|---|---|---|---|---|---|---|---|---|
| 375% | 200% | 2.0 | 150% | 2.0 | 100 % | 2.0 | 60% | 5.0 |
| 370% | 200% | 2.0 | 150% | 2.0 | 100% | 2.0 | 60% | 5.0 |
| 365% | 195% | 2.0 | 150% | 2.0 | 100% | 2.0 | 60% | 5.0 |
| 360% | 195% | 2.0 | 145% | 2.0 | 100% | 2.0 | 60% | 5.0 |
| 355% | 190% | 2.0 | 145% | 2.0 | 95% | 2.0 | 60% | 5.0 |
| 350% | 190% | 2.0 | 140% | 2.0 | 95% | 2.0 | 60% | 5.0 |
| 345% | 185% | 2.0 | 140% | 2.0 | 95% | 2.0 | 60% | 5.0 |
| 340% | 185% | 2.0 | 140% | 2.0 | 95% | 2.0 | 55% | 5.0 |
| 335% | 180% | 2.0 | 135% | 2.0 | 90% | 2.0 | 55% | 5.0 |
| 330% | 180% | 2.0 | 135% | 2.0 | 90% | 2.0 | 55% | 5.0 |
| 325% | 175% | 2.0 | 130% | 2.0 | 90% | 2.0 | 55% | 5.0 |
| 320% | 175% | 2.0 | 130% | 2.0 | 90% | 2.0 | 55% | 5.0 |
| 315% | 170% | 2.0 | 130% | 2.0 | 85% | 2.0 | 55% | 5.0 |
| 310% | 170% | 2.0 | 125% | 2.0 | 85% | 2.0 | 50% | 5.0 |
| 305% | 165% | 2.0 | 125% | 2.0 | 85% | 2.0 | 50% | 5.0 |
| 300% | 160% | 2.0 | 120% | 2.0 | 80% | 2.0 | 50% | 5.0 |
| 295% | 160% | 2.0 | 120% | 2.0 | 80% | 2.0 | 50% | 5.0 |
| 290% | 155% | 2.0 | 120% | 2.0 | 80% | 2.0 | 50% | 5.0 |
| 285% | 155% | 2.0 | 115% | 2.0 | 80% | 2.0 | 50% | 5.0 |
| 280% | 150% | 2.0 | 115% | 2.0 | 75% | 2.0 | 45% | 5.0 |
| 275% | 150% | 2.0 | 110% | 2.0 | 75% | 2.0 | 45% | 5.0 |
| 270% | 145% | 2.0 | 110% | 2.0 | 75% | 2.0 | 45% | 5.0 |
| 265% | 145% | 2.0 | 110% | 2.0 | 75% | 2.0 | 45% | 5.0 |
| 260% | 140% | 2.0 | 105% | 2.0 | 70% | 2.0 | 45% | 5.0 |
| 255% | 140% | 2.0 | 105% | 2.0 | 70% | 2.0 | 45% | 5.0 |
| 250% | 135% | 2.0 | 100% | 2.0 | 70% | 2.0 | 40% | 5.0 |
| 245% | 135% | 2.0 | 100% | 2.0 | 70% | 2.0 | 40% | 5.0 |
| 240% | 130% | 2.0 | 100% | 2.0 | 65% | 2.0 | 40% | 5.0 |
| 235% | 130% | 2.0 | 95% | 2.0 | 65% | 2.0 | 40% | 5.0 |
| 230% | 125% | 2.0 | 95% | 2.0 | 65% | 2.0 | 40% | 5.0 |

(continued)

| Peak Percent Strain | First Hold Percent Strain (Steps 4 and 5) | First Hold Time (Minutes) (Step 5) | Second Hold Percent Strain (Steps 6 and 7) | Second Hold Time (Minutes) (Step 7) | Third Hold Percent Strain (Steps 8 and 9) | Third Hold Time (Minutes) (Step 9) | Fourth Hold Percent Strain (Steps 10 and 11) | Fourth Hold Time (Minutes) (Step 11) |
|---|---|---|---|---|---|---|---|---|
| 225% | 120% | 2.0 | 90% | 2.0 | 60% | 2.0 | 40% | 5.0 |
| 220% | 120% | 2.0 | 90% | 2.0 | 60% | 2.0 | 40% | 5.0 |
| 215% | 115% | 2.0 | 90% | 2.0 | 60% | 2.0 | 35% | 5.0 |
| 210% | 115% | 2.0 | 85% | 2.0 | 60% | 2.0 | 35% | 5.0 |
| 205% | 110% | 2.0 | 85% | 2.0 | 55% | 2.0 | 35% | 5.0 |
| 200% | 110% | 2.0 | 80% | 2.0 | 55% | 2.0 | 35% | 5.0 |
| 195% | 105% | 2.0 | 80% | 2.0 | 55% | 2.0 | 35% | 5.0 |
| 190% | 105% | 2.0 | 80% | 2.0 | 55% | 2.0 | 35% | 5.0 |
| 185% | 100% | 2.0 | 75% | 2.0 | 50% | 2.0 | 30% | 5.0 |
| 180% | 100% | 2.0 | 75% | 2.0 | 50% | 2.0 | 30% | 5.0 |
| 175% | 95% | 2.0 | 70% | 2.0 | 50% | 2.0 | 30% | 5.0 |
| 170% | 95% | 2.0 | 70% | 2.0 | 50% | 2.0 | 30% | 5.0 |
| 165% | 90% | 2.0 | 70% | 2.0 | 45% | 5.0 | 30% | 5.0 |
| 160% | 90% | 2.0 | 65% | 2.0 | 45% | 5.0 | 30% | 5.0 |
| 155% | 85% | 2.0 | 65% | 2.0 | 45% | 5.0 | 25% | 5.0 |
| 150% | 80% | 2.0 | 60% | 2.0 | 40% | 5.0 | 25% | 5.0 |
| 145% | 80% | 2.0 | 60% | 2.0 | 40% | 5.0 | 25% | 5.0 |
| 140% | 75% | 2.0 | 60% | 2.0 | 40% | 5.0 | 25% | 5.0 |
| 135% | 75% | 2.0 | 55% | 2.0 | 40% | 5.0 | 25% | 5.0 |
| 130% | 70% | 2.0 | 55% | 2.0 | 35% | 5.0 | 25% | 5.0 |
| 125% | 70% | 2.0 | 50% | 2.0 | 35% | 5.0 | 20% | 5.0 |
| 120% | 65% | 2.0 | 50% | 2.0 | 35% | 5.0 | 20% | 5.0 |
| 115% | 65% | 2.0 | 50% | 2.0 | 35% | 5.0 | 20% | 5.0 |
| 110% | 60% | 2.0 | 45% | 5.0 | 30% | 5.0 | 20% | 5.0 |
| 105% | 60% | 2.0 | 45% | 5.0 | 30% | 5.0 | 20% | 5.0 |
| 100% | 60% | 2.0 | 45% | 5.0 | 30% | 5.0 | 20% | 5.0 |
| 95% | 60% | 2.0 | 45% | 5.0 | 30% | 5.0 | 20% | 5.0 |
| 90% | 60% | 2.0 | 45% | 5.0 | 30% | 5.0 | 20% | 5.0 |
| 85% | 60% | 2.0 | 45% | 5.0 | 30% | 5.0 | 20% | 5.0 |
| 80% | 60% | 2.0 | 45% | 5.0 | 30% | 5.0 | 20% | 5.0 |
| 75% | 60% | 2.0 | 45% | 5.0 | 30% | 5.0 | 20% | 5.0 |
| 70% | 60% | 2.0 | 45% | 5.0 | 30% | 5.0 | 20% | 5.0 |
| 65% | 60% | 2.0 | 45% | 5.0 | 30% | 5.0 | 20% | 5.0 |
| 60% | 60% | 2.0 | 45% | 5.0 | 30% | 5.0 | 20% | 5.0 |

(continued)

| Peak Percent Strain | First Hold Percent Strain (Steps 4 and 5) | First Hold Time (Minutes) (Step 5) | Second Hold Percent Strain (Steps 6 and 7) | Second Hold Time (Minutes) (Step 7) | Third Hold Percent Strain (Steps 8 and 9) | Third Hold Time (Minutes) (Step 9) | Fourth Hold Percent Strain (Steps 10 and 11) | Fourth Hold Time (Minutes) (Step 11) |
|---|---|---|---|---|---|---|---|---|
| 55% | 55% | 2.0 | 45% | 5.0 | 30% | 5.0 | 15% | 5.0 |
| 50% | 50% | 2.0 | 40% | 5.0 | 25% | 5.0 | 15% | 5.0 |
| 45% | 45% | 5.0 | 35% | 5.0 | 25% | 5.0 | 10% | 5.0 |
| 40% | 40% | 5.0 | 30% | 5.0 | 20% | 5.0 | 10% | 5.0 |
| 35% | 35% | 5.0 | 30% | 5.0 | 20% | 5.0 | 10% | 5.0 |
| 30% | 30% | 5.0 | 25% | 5.0 | 15% | 5.0 | 5% | 5.0 |
| 25% | 25% | 5.0 | 20% | 5.0 | 15% | 5.0 | 5% | 5.0 |
| 20% | 20% | 5.0 | 15% | 5.0 | 10% | 5.0 | 5% | 5.0 |
| 15% | 15% | 5.0 | 10% | 5.0 | 5% | 5.0 | Skip | Skip |
| 10% | 10% | 5.0 | 5% | 5.0 | Skip | Skip | Skip | Skip |
| 5% | 5% | 5.0 | Skip | Skip | Skip | Skip | Skip | Skip |

OIL EXPOSURE METHOD

This method is used to determine the effect of exposing a slow recovery stretch laminate comprising an elastic member or a slow recovery elastomer to an excess of mineral oil and separately to an excess of isopropyl palmitate, a common ingredient in many baby oils, lotions, gels, cremes, and the like. A small sample (~0.1 grams) of either a stretch laminate or an elastic member is placed in each of two clean vials. To one vial is added about 10 ml of mineral oil (Britol® 50T available from Crompton Corporation, Petrolia, PA), and to a second vial is added about 10 ml of isopropyl palmitate (90+% grade available from Sigma-Aldrich, St. Louis, MO). It may be necessary to gently mix the contents in order to completely wet or submerge the sample into the mineral oil or isopropyl palmitate. The mixtures are allowed to sit for 30 hours at room temperature (22°C). After 30 hours, a visual observation is made of each sample, and if any sample appears to remain intact, it is removed from the vial and its ability to be extended is approximated by hand, e.g., when clamped between the thumb and forefinger on each hand and then pulled apart. If the samples remain intact after 30 hours in both mineral oil and isopropyl palmitate, and if the samples after the 30 hours maintain an extension of greater than about 50% engineering strain from both mineral oil and isopropyl palmitate, then the laminate or elastomer samples are considered to have passed the Oil Exposure Method. Other mineral oils are within the scope of this method including commercial baby oils that list mineral oil as the primary ingredient, e.g., Johnson's Baby Oil which is available in the U.S. from Johnson & Johnson, New Brunswick, NJ.

EXAMPLES

EXAMPLE 1. Polymer Molecular Weight Determination

Polymer number average molecular weight and molecular weight distributions are determined by GPC SEC/MALS. The GPC uses a Waters Alliance 2695 HPLC autoinjector. It contains three Styragel HR columns (HR3, HR4 and HR5). The column heater is set to 30°PC. The flow rate is 1.0 mL/min and the mobile phase is tetrahydrofuran, HPLC grade available from Sigma-Aldrich Inc., St. Louis, MO. The detectors are a Wyatt Dawn EOS Light scattering detector calibrated with toluene and normalized using 100 kilo Dalton polystyrene (molecular weight standard available from Polysciences, Inc., Warrington, PA) in mobile phase and a Waters 2414 refractive index detector at 30°C. Samples for analysis are prepared at a known concentration of 2 mg/mL. Samples are filtered using 0.45$\mu$m nylon membrane filters. The injection volume is 100$\mu$l. The data is collected and analyzed using ASTRA 5.3.2.15. Values for $dn/dc$ are calculated from the refractive index trace assuming 100% mass recovery.

EXAMPLE 2. Styrene Monomer Purification

Styrene (available from Sigma-Aldrich) is purified by passing through an activated alumina (available from Sigma-Aldrich) column under nitrogen atmosphere to remove inhibitors and then the styrene is added to a clean, dry round bottom flask filled with nitrogen and fitted with a rubber septum.

EXAMPLE 3. Randomization Catalyst

A randomization catalyst is generated by the reaction of 1 gram of potassium metal (available from Sigma-Aldrich) with 1.16 grams of 2,3-dimethyl-3-pentanol (available from Sigma-Aldrich) dissolved in 50ml of cyclohexane (PRA grade available from Sigma-Aldrich).

EXAMPLE 4. Purification of t-butyl styrene

Tert-butyl styrene (available from Sigma-Aldrich) is purified by passing through an activated alumina (available from Sigma-Aldrich) column under nitrogen atmosphere to remove inhibitors and then the t-butyl styrene is added to a clean, dry round bottom flask filled with nitrogen and fitted with rubber septa.

EXAMPLE 5. Hydrogenation Catalyst

Hydrogenation catalyst is prepared as follows; 0.345 grams of nickel(2-ethyl hexanoate) (0.001 mole) (available from Sigma-Aldrich) is dissolved in 30 ml of cylcohexane (PRA grade available from Sigma-Aldrich). To this is added 3 ml of triethylaluminum (0.003mole) (1.0M in hexanes available from Sigma-Aldrich) resulting in a black dispersion of nickel catalyst.

EXAMPLE 6. Preparation of acetyl nitrate

A solution of acetyl nitrate is prepared as follows. To 600 ml of methylene chloride (available from Sigma-Aldrich) is added 320 grams of acetic anhydride (available from Sigma-Aldrich) and this is cooled to (0°C. To this is slowly added 100 grams of nitric acid (available from Sigma-Aldrich), while maintaining the temperature at (0°C. This is allowed to react for 60 minutes at (0°C.

EXAMPLE 7. Synthesis of Polystyrene Block Copolymer with Isoprene Soft Block

To a clean reactor at 60°C is added 3 liters of cyclohexane (pesticide residue analysis (PRA) grade from Sigma Aldrich) and 60 grams of styrene (as purified in Example 2). This is titrated with s-butyl lithium (available from Sigma-Aldrich) to a persistent yellow color and 5 mmole of s-butyl lithium is added to give the desired molecular weight. After 20 minutes, a sample is taken and 280 grams of isoprene (available from Sigma-Aldrich) is added to the reactor. This is allowed to react for 45 minutes maintaining the temperature at 60°C. A sample is taken for analysis and 60 grams of styrene (as purified in Example 2) is added. After 20 minutes the reaction is terminated with degassed methanol. A sample is taken for analysis, stabilized with 0.1 weight percent Irganox 1010 (available from BASF), and vacuum dried.
The polymer solution is precipitated by pouring it into a large excess of methanol with vigorous stirring. The polymer precipitate is filtered and Irganox 1010 is added to stabilize the polymer which is then vacuum dried.
GPC analysis by the method of Example 1 shows the 1st block with a number average molecular weight of Mn=12.8 kilo Daltons and a molecular weight distribution of Mw/Mn=1.08, the final triblock with a number average molecular weight of 80.0 kilo Daltons and a molecular weight distribution of Mw/Mn=1.02, and an overall composition of 27 weight percent styrene and 73 weight percent isoprene.

EXAMPLE 8. Hydrogenation of Polystyrene Block Copolymer with Isoprene Soft Block

300 grams of the polymer from Example 7 is dissolved in 2500 ml of cyclohexane (pesticide residue analysis (PRA) grade from Sigma-Aldrich). This solution is degassed by bubbling nitrogen thru it for two minutes. This solution is added to a pressure reactor and the hydrogenation catalyst from Example 5 is added and 50 psi of hydrogen pressure is maintained while providing vigorous stirring (500 rpm). The hydrogenation is carried out for 16 hours at which point the polymer solution is removed from the reactor into ajar with a solution of 1 liter of 0.5 molar HCl. This is mixed with vigorous agitation until the black catalyst is oxidized and the polymer solution becomes clear. The mixture is allowed to settle into two layers and the water layer is discarded. To the polymer/cyclohexane solution is added 1 liter of 0.5M aqueous sodium hydroxide solution (available from Sigma-Aldrich). This is mixed vigorously for 5 minutes and then allowed to settle into two layers. The aqueous layer is discarded and the polymer/cyclohexane layer is stabilized with 0.1 weight percent Irganox 1010 (available from BASF) and the polymer solution is then dried to isolate the polymer. NMR analysis of the dried polymer shows about 100% hydrogenation of the isoprene double bonds.

EXAMPLE 9. Nitration of Polystyrene Block Copolymer with Ethylene/Propylene Soft Block

50 grams of the polymer from Example 8 is dissolved in 500 ml of methylene chloride (available from Sigma-Aldrich) at 0°C, along with 20 ml of acetic anhydride (available from Sigma-Aldrich) to which is added 129 ml of the acetyl nitrate from Example 6. This mixture is reacted for 120 minutes at 0°C, and the reaction is stopped by precipitation of the solution into 3 liters of methanol. Elemental analysis shows a 30% degree of nitration.

EXAMPLE 10. Synthesis of Polystyrene Block Copolymer with Random Styrene-Isoprene Soft Block

To a clean reactor at 60°C is added 3 liters of cyclohexane (pesticide residue analysis (PRA) grade from Sigma-Aldrich) and 60 grams of styrene (as purified in Example 2). This is titrated with s-butyl lithium (available from Sigma-Aldrich) to a persistent yellow color and 5 mmole of s-butyl lithium is added to give the desired molecular weight. This is followed by addition of 0.2 mole (1ml) of the randomization catalyst from Example 3. After 20 minutes, a sample is taken and 95 grams of isoprene (available from Sigma-Aldrich) and 85 grams of styrene (as purified in Example 9) is added to the reactor. This is allowed to react for 45 minutes maintaining the temperature at 50°C. A sample is taken for analysis and 40 grams of styrene (as purified in Example 2) is added. After 20 minutes the reaction is terminated with degassed methanol. A 20 gram sample is taken for analysis and testing, stabilized with 0.1 weight percent Irganox 1010 (available from BASF), and vacuum dried.
GPC analysis of the final triblock by the method of Example 1 shows a number average molecular weight of Mn=65 kilo Daltons and a molecular weight distribution of Mw/Mn=1.03.

EXAMPLE 11. Hydrogenation of Polystyrene Block Copolymer with Random Styrene-Isoprene Soft Block

150 grams of the polymer from Example 10 is dissolved in 2500 ml of cyclohexane (pesticide residue analysis (PRA) grade from Sigma-Aldrich). This solution is degassed by bubbling nitrogen thru it for two minutes. This solution is added to a pressure reactor and the hydrogenation catalyst from Example 5 is added and 50 psi of hydrogen pressure is maintained while providing vigorous stirring (500rpm). The hydrogenation is carried out for 16 hours at which point the polymer solution is removed from the reactor into a jar with a solution of 1 liter of 0.5 molar HCl. This is mixed with vigorous agitation until the black catalyst is oxidized and the polymer solution becomes clear. The mixture is allowed to settle into two layers and the water layer is discarded. To the polymer/cyclohexane solution is added 1 liter of 0.5M aqueous sodium hydroxide solution. This is mixed vigorously for 5 minutes and then allowed to settle into two layers. The aqueous layer is discarded and the polymer/cyclohexane layer is stabilized with 0.1 weight percent Irganox 1010 (available from BASF) and the polymer solution is then dried to isolate the polymer. NMR analysis of the dried polymer shows about 100% hydrogenation of the isoprene double bonds.

EXAMPLE 12. Nitration of Polystyrene Block Copolymer with Random Styrene-Ethylene/Propylene Soft Block

50 grams of the polymer from Example 11 is dissolved in 500 ml of methylene chloride (available from Sigma-Aldrich) at 0°C, to which is added 20 ml of acetic anhydride (available from Sigma-Aldrich) and 129 ml of the acetyl nitrate from Example 6. This mixture is reacted for 120 minutes at 0°C, and the reaction is stopped by precipitation of the solution into 3 liters of methanol. The precipitate is further washed with a 50/50 by volume ethanol/water solution then soaked in water overnight. The polymer is then washed with ethanol and vacuum dried.

EXAMPLE 13. Synthesis of Polystyrene Block Copolymer with Random t-Butyl Styrene-Isoprene Soft Block

To a clean reactor at 20°C, is added 3 liters of cyclohexane (pesticide residue analysis (PRA) grade from Sigma-Aldrich), 2 ml of tetrahydrofuran (available from Sigma-Aldrich) and 60 grams of styrene (as purified in Example 2). This is titrated with s-butyl lithium (available from Sigma-Aldrich) to a persistent yellow color and 5 mmole of s-butyl lithium is added to give the desired molecular weight. After 20 minutes, a sample is taken and 132 grams of isoprene (available from Sigma-Aldrich) along with 55 grams of purified t-butyl styrene (as prepared in Example 4) are added to the reactor. This is allowed to react for 180 minutes maintaining the temperature at 30°C. A sample is taken for analysis and 40 grams of styrene (as purified in Example 2) is added. After 20 minutes the reaction is terminated with degassed methanol. A 20 gram sample is taken for analysis and testing, stabilized with 0.1 weight percent Irganox 1010 (available from BASF), and vacuum dried.

GPC analysis of the final triblock by the method of Example 1 shows a number average molecular weight of Mn=80 kilo Daltons and a molecular weight distribution of Mw/Mn=1.01.

EXAMPLE 14. Hydrogenation of Polystyrene Block Copolymer with Random t-Butyl Styrene-Isoprene Soft Block

200 grams of the polymer from Example 13 is dissolved in 2500 ml of cyclohexane (pesticide residue analysis (PRA) grade from Sigma-Aldrich). This solution is degassed by bubbling nitrogen thru it for two minutes. This solution is added to a pressure reactor and the hydrogenation catalyst from Example 5 is added and 50 psi of hydrogen pressure is maintained while providing vigorous stirring (500rpm). The hydrogenation is carried out for 16 hours at which point the polymer solution is removed from the reactor into a jar with a solution of 1 liter of 0.5 molar HCl. This is mixed with vigorous agitation until the black catalyst is oxidized and the polymer solution becomes clear. The mixture is allowed to settle into two layers and the water layer is discarded. To the polymer/cyclohexane solution is added 1 liter of 0.5M aqueous sodium hydroxide solution. This is mixed vigorously for 5 minutes and then allowed to settle into two layers. The aqueous layer is discarded and the polymer/cyclohexane layer is stabilized with 0.1 weight percent Irganox 1010 (available from BASF) and the polymer solution is then dried to isolate the polymer. NMR analysis of the dried polymer shows about 100% hydrogenation of the isoprene double bonds.

EXAMPLE 15. Nitration of Polystyrene Block Copolymer with Random t-Butyl Styrene-Ethylene/Propylene Soft Block

50 grams of the polymer from Example 14 is dissolved in 500ml of methylene chloride (available from Sigma-Aldrich) at 0°C, to which is added 50ml of acetic anhydride (available from Sigma-Aldrich) and 138 ml of the acetyl nitrate prepared in Example 6. This mixture is reacted for 120 minutes at 0°C, and the reaction is stopped by precipitation of the solution into 5 liters of ethanol. The precipitate is further washed with ethanol and vacuum dried overnight.

EXAMPLE 16. Nitration of Polystyrene Block Copolymer with Random Ethylene-Ethylene/Propylene Soft Block

25.4 grams of Septon 4033 (available from Kuraray America Inc., Pasedena, TX) is dissolved in 500 ml of methylene chloride (available from Sigma-Aldrich) at 0°C. Separately, 12.66 grams of chlorine is condensed into 52.75 grams of methylene chloride. 12.66 grams of this chlorine solution is added into the Septon solution and allowed to mix at room temperature (22°C). After two days of reaction, 15ml of acetic anhydride is added to the Septon solution and this is allowed to stir for one hour. A separate solution of 100 ml of methylene chloride and 30.24 grams of acetic anhydride is made and cooled to 0°C and then 13.1 grams of nitric acid is added slowly. This acetyl-nitrate solution is added drop wise to the Septon solution which is cooled to -10°C. The entire reaction is kept in the dark. After two hours at -10°C, the reaction is allowed to warm to 0°C for an additional hour of reaction, then it is precipitated into 3 liters of methanol. This mixture is filtered and washed with 50/50 by volume ethanol/water, then soaked in water overnight. The next day this is filtered and washed with ethanol and then vacuum dried.

GPC analysis by the method of Example 1 shows a number average molecular weight of Mn=85 kilo Daltons and a molecular weight distribution of Mw/Mn=1.09. Elemental analysis shows a 32% degree of nitration.

EXAMPLE 17. Chlorination of Polystyrene Block Copolymer with Ethylene-Ethylene /Propylene Soft Block

50 grams of Septon 4033 (available from Kuraray America Inc., Pasedena, TX) is dissolved in 500 ml of methylene chloride (available from Sigma-Aldrich). Separately, 12 grams of chlorine ( available from Sigma-Aldrich) is condensed into 52.75 grams of methylene chloride. The Septon solution is cooled to 0°C in a flask without light, and 2 grams of iron filings (available from Sigma-Aldrich) are added and then the solution containing the 12 grams of chlorine is added over a 30 minute period. This mixture is reacted for an additional 30 minutes at 0°C, then the reaction is stopped by precipitation of the solution into 3 liters of methanol. The precipitate is further washed with a 50/50 by volume ethanol/water solution then soaked in water overnight. The polymer is then washed with ethanol and vacuum dried.

EXAMPLE 18. Bromination of Polystyrene Block Copolymer with Ethylene-Ethylene /Propylene Soft Block (Prophetic)

50 grams of Septon 4033 (available from Kuraray America Inc., Pasedena, TX) is dissolved in 500 ml of methylene chloride (available from Sigma-Aldrich) and this is cooled to 0°C and kept in the dark. 2 grams of iron filings (available from Sigma-Aldrich) is added to the Septon solution and 25 grams of bromine (available from Sigma-Aldrich) is added dropwise over one hour.

HBr generated from the reaction is trapped by purging the nitrogen thru sodium bicarbonate. This mixture is reacted for 30 minutes at 0°C, then the reaction is heated to 50°C for one hour and the reaction is stopped by precipitation of the solution into 3 liters of methanol. The precipitate is further washed with a 50/50 by volume ethanol/water solution then soaked in water overnight. The polymer is then washed with ethanol and vacuum dried.

EXAMPLE 19. Preparation of a Methyl Ester Modified Polystyrene Block Copolymer with an Ethylene-Ethylene /Propylene Soft Block (Prophetic)

50 grams of Septon 4033 (available from Kuraray America Inc., Pasedena, TX) dissolved in 500 ml of cyclohexane (available from Sigma-Aldrich). This solution is cooled to 0°C and 20 grams of trichloroaluminum (available from Sigma-Aldrich) is added. 20 grams of methylchloroacetate (available from Sigma-Aldrich) is added dropwise over 30 minutes. After 60 minutes at 0°C, the reaction is heated at 50°C for 1 hour then the product is isolated by pouring into water at 0°C. The solids are washed in water to pH 7, and then washed with ethanol and vacuum dried.

EXAMPLE 20. Preparation of a Benzyl Ester Modified Polystyrene Block Copolymer with an Ethylene-Ethylene /Propylene Soft Block (Prophetic)

50 grams of Septon 4033 (available from Kuraray America Inc., Pasedena, TX) dissolved in 500 ml of cyclohexane (available from Sigma-Aldrich). This solution is cooled to 0°C and 20 grams of trichloroaluminum (available from Sigma-Aldrich) is added. 35 grams of benzyl-chloroacetate (available from Sigma-Aldrich) is added dropwise over 30 minutes. After 60 minutes at 0°C, the reaction is heated at 5°C for 1 hour then the product is isolated by pouring into water at 0°C. The solids are washed in water to pH 7 and then washed with ethanol and vacuum dried.

EXAMPLE 21. Aromatic Substitution of Nitrile groups onto a Polystyrene Block Copolymer with an Ethylene-Ethylene /Propylene Soft Block (Prophetic)

50 grams of Septon 4033 (available from Kuraray America Inc., Pasedena, TX) is dissolved in 500 ml of carbon disulfide (available from Sigma-Aldrich) at 0°C. To this is added 35grams of BrCN (available from Sigma-Aldrich) and then 250grams of anhydrous aluminum trichloride (available from Sigma-Aldrich) is added in small portions over the period of an hour. The mixture is refluxed for 24 hours and then poured into water/dichloromethane and washed with water.

EXAMPLE 22. Aromatic Substitution of a Methyl Ketone onto a Polystyrene Block Copolymer with an Ethylene-Ethylene /Propylene Soft Block (Prophetic)

50 grams of Septon 4033 (available from Kuraray America Inc., Pasedena, TX) is dissolved in 500 ml of cyclohexane (available from Sigma-Aldrich) at 25°C The solution is placed in a round bottom flask with condenser and stirrer and the outlet of the condenser is connected to a trap for absorbing the hydrogen chloride generated by the reaction. To this solution is added 26.7grams of anhydrous aluminum chloride (available from Sigma-Aldrich) and then 11.8grams of Acetyl chloride (available from Sigma-Aldrich) is added drop-wise over a 1 hour period. This mixture is then heated to 50°C and reacted for 120 minutes. The reaction is stopped by precipitation of the solution into 3 liters of water. The precipitate is further washed with methanol solution then soaked in water overnight. The polymer is then washed with ethanol and vacuum dried.

EXAMPLE 23. Aromatic Substitution of a Butyl Ketone onto a Polystyrene Block Copolymer with an Ethylene-Ethylene /Propylene Soft Block (Prophetic)

50 grams of Septon 4033 (available from Kuraray America Inc., Pasedena, TX) is dissolved in 500 ml of cyclohexane (available from Sigma-Aldrich) at 25°C The solution is placed in a round bottom flask with condenser and stirrer and the outlet of the condenser is connected to a trap for absorbing the hydrogen chloride generated by the reaction. To this solution is added 26.7grams of anhydrous aluminum chloride (available from Sigma-Aldrich) and then 18.1 grams of butanoyl chloride (Valeroyl chloride) (available from Sigma-Aldrich) is added drop-wise over a 1 hour period. This mixture is then heated to 50°C and reacted for 120 minutes. The reaction is stopped by precipitation of the solution into 3 liters of water. The precipitate is further washed with methanol solution then soaked in water overnight. The polymer is then washed with ethanol and vacuum dried.

EXAMPLE 24. Aromatic Substitution of a Phenyl Ketone onto a Polystyrene Block Copolymer with an Ethylene-Ethylene /Propylene Soft Block (Prophetic)

50 grams of Septon 4033 (available from Kuraray America Inc., Pasedena, TX) is dissolved in 500 ml of cyclohexane (available from Sigma-Aldrich) at 25°C The solution is placed in a round bottom flask with condenser and stirrer and the outlet of the condenser is connected to a trap for absorbing the hydrogen chloride generated by the reaction. To this solution is added 26.7grams of anhydrous aluminum chloride (available from Sigma- Aldrich) and then 21.1 grams of Benzoyl chloride (available from Sigma- Aldrich) is added drop- wise over a 1 hour period. This mixture is then heated to 50C and reacted for 120 minutes. The reaction is stopped by precipitation of the solution into 3 liters of water. The precipitate is further washed with methanol solution then soaked in water overnight. The polymer is then washed with ethanol and vacuum dried.

EXAMPLE 25.

An elastomer composition consisting of 45.1 wt% of the polymer from Example 16, 53.0 wt% Eastotac H-142R modifying resin (available from Eastman Chemical Company, Kingsport, TN), and 1.9 wt% Britol(R) 50T, mineral oil (available from Crompton Corporation, Petrolia, PA) is prepared by solution blending and compression molding into films. This method consists of- (1) dissolving all components in a suitable solvent, e.g. , dichloromethane (available from Sigma- Aldrich), where the weight percent of solids is about 5%, (2) pouring the solution into a petri- dish, or other suitable container, and allowing to dry overnight at room temperature, (3) vacuum drying the films at about 12CPC for about 1 hour, and (4) preparing compression molded films. A compression molding press with 9 inch (22.86 cm) by 9 inch (22.86 cm) heated platens (Carver, Inc., Wabash, IN, Model number 3853-0/3925) is used along with a custom mold to prepare compression molded film samples. The mold is an assembly of two metal plates (about 9 inches (22.86 cm) wide and about 12 inches (30.48 cm) long), two sheets of Teflon(R) film (about 6 inches (15.24 cm) wide, about 12 inches (30.48 cm) long, and about 0.010 inches (0.25 mm) thick), and two metal shims (about 1 inch (2.54 cm) wide, about 12 inches (30.48 cm) long, and about 0.027 (0.69mm) inches thick). The assembly forms a mold about 0.007 (0.018 mm) inches deep. The metal platens on the compression molding press are set to a temperature of 18CPC and the metal plates are preheated by stacking them onto the lower platen. After the temperature of the platens and metal plates has reached the set temperature, about 3 grams of the vacuum dried composition is placed on one of the Teflon(R) sheets, which is then placed on top of one of the preheated metal plates. The shims are placed on the outer edges of the metal plate, outside of the Teflon(R) sheets. The second Teflon(R) sheet and second preheated metal plate are then placed on top to finish assembly of the mold. The entire mold assembly is placed in the compression molding press and maintained at the set temperature with about 1000 pounds per square inch (178.6 kg/cm$^2$) of pressure on the mold. After about 30 seconds, the pressure applied to the mold is increased to about 10,000 pounds per square inch (1785.8 kg/cm$^2$) and held for about 30 seconds. The pressed film, maintained between the Teflon(R) sheets, is removed from the mold and cooled to room temperature (about 2 C).The film is removed from between the Teflon(R) sheets, folded and repressed according to the procedure outlined above with the exception that after the about 30 second hold at 1000 pounds per square inch (178.6 kg/cm$^2$), about 15,000 pounds per square inch (2678.7 kg/cm$^2$) is applied to the mold and is held for about 45 seconds. The film is removed from the mold after the second press, maintained between the Teflon(R) sheets, and cooled to room temperature (about 22C). The film is removed from between the Teflon(R) sheets, and stored at room temperature between double sided release paper for about 2 days before evaluating the properties of the film. A minimum of five random samples are cut and tested from at least two different compression molded films, and the number of test samples are split relatively evenly between the number of compression molded films. For example, if two compression molded film samples are produced, 3 random test samples are taken from one molded film and 2 random test samples are taken from the other molded film.

Films of this elastomer composition are measured according to the Post Elongation Recovery and show a percent of initial strain after 15 seconds recovery at 22C of 39%.

EXAMPLE 26.

Samples of the following materials are tested according to the Oil Exposure method described in the Test Methods section using Johnson's Baby Oil (available in the U.S. from Johnson & Johnson, New Brunswick, NJ where mineral oil and fragrance are the listed ingredients) and isopropyl palmitate (90+% available from Sigma- Aldrich): (1) elastomer film from Example 25, and (3) elastomer resin Septon 4033 (available from Kuraray America Inc., Pasedena, TX). After 30 hours exposure to excess baby oil at room temperature, both materials are swollen with oil and remain intact, and the elastomer film from Example 25 is readily discernable by hand to be stretchable to at least about 500% engineering strain. After 30 hours exposure to excess isopropyl palmitate at room temperature, the Septon 4033 resin is completely dissolved while the elastomer film from Example 25 remains intact and is readily discernable by hand to be stretchable to at least about 100% engineering strain.

EXAMPLE 27. (Prophetic)

Table 2 shows the solubility parameters of various substituted polystyrenes, and Tables 3 and 4 show the solubility parameters of various random copolymers of styrene and substituted styrene. The solubility parameters are determined according to the method described by L.H.

Sperling in <u>Introduction to Physical Polymer Science,</u> Wiley-Interscience (New York, 1992). Additionally, according to the method described by Sperling, the solubility parameter of polystyrene is 8.96 $(cal/cm^3)^{1/2}$, the solubility parameter of isopropyl palmitate is 8.12 $(cal/cm^3)^{1/2}$, and the solubility parameter of mineral oil (dodecane) is 7.75 $(cal/cm^3)^{1/2}$, where the densities used in determining these solubility parameters are 1.04 $g/cm^3$, 0.85 $g/cm^3$, and 0.75 $g/cm^3$, respectively.

Table 2. Solubility Parameters of Substituted Polystyrenes

| Chemical Name | Chemical Abbreviation* | Solubility Parameter [$(cal/cm^3)^{1/2}$] | | |
|---|---|---|---|---|
| | | Monofunctional** | Difunctional** | Trifunctional** |
| Nitro substituted polystyrene | NO2-PS | 9.86 (1.1) | 10.07 (1.1) | 10.20 (1.1) |
| Chlorine substituted polystyrene | Cl-PS | 10.10 (1.2) | 10.79 (1.3) | 11.92 (1.46) |
| Bromine substituted polystyrene | Br-PS | 10.14 (1.5) | 11.74 (1.95) | 12.93 (2.3) |
| Nitrile substituted polystyrene | CN-PS | 11.14 (1.1) | 12.26 (1.1) | 13.06 (1.1) |
| Methyl ketone substituted polystyrene | Me-CO-PS | 9.49 (1.0) | --- | --- |
| Ethyl ketone substituted polystyrene | Et-CO-PS | 9.49 (1.0) | --- | --- |
| Propyl ketone substituted polystyrene | Pr-CO-PS | 9.49 (1.0) | --- | --- |
| Butyl ketone, substituted polystyrene | Bu-CO-PS | 9.39 (0.99) | --- | --- |
| Pentyl ketone substituted polystyrene | Pen-CO-PS | 9.06 (0.96) | --- | --- |
| Hexyl ketone substituted polystyrene | Hex-CO-PS | 8.92 (0.94) | --- | --- |
| Phenyl ketone substituted polystyrene | Ph-CO-PS | 9.16 (1.0) | --- | --- |
| Methyl acetylester substituted polystyrene | Me-COO-PS | 9.71 (1.1) | 9.76 (1.1) | 9.79 (1.1) |
| Butyl acetylester substituted polystyrene | Bu-COO-PS | 9.58 (1.07) | 9.67 (1.07) | 9.71 (1.07) |
| Hexyl acetylester substituted polystyrene | Hex-COO-PS | 9.38 (1.04) | 9.46 (1.04) | 9.51 (1.04) |
| Phenyl acetylester substituted polystyrene | Ph-COO-PS | 10.02 (1.15) | 10.03 (1.15) | 10.03 (1.15) |
| Benzyl acetylester substituted polystyrene | Ben-COO-PS | 10.51 (1.2) | 10.53 (1.2) | 10.54 (1.2) |
| *PS= Polystyrene <br> **Number in parenthesis is the density in $g/cm^3$ used in determining the solubility parameter | | | | |

Table 3. Solubility Parameters of Random Copolymers of Styrene and Substituted Styrene (Part 1)

| Degree of Substitution (%) | Solubility Parameter [$(cal/cm^3)^{1/2}$] | | | | |
|---|---|---|---|---|---|
| | PS-co-NO2-PS | PS-co-Cl-PS | PS-co-Br-PS | PS-co-CN-PS | PS-co-Ph-CO-PS |
| 0 | 8.96 | 8.96 | 8.96 | 8.96 | 8.96 |
| 10 | 9.05 | 9.07 | 9.08 | 9.18 | 8.98 |

(continued)

| Degree of Substitution (%) | Solubility Parameter [(cal/cm³)^{1/2}] | | | | |
|---|---|---|---|---|---|
| | PS-co-NO2-PS | PS-co-Cl-PS | PS-co-Br-PS | PS-co-CN-PS | PS-co-Ph-CO-PS |
| 20 | 9.14 | 9.19 | 9.20 | 9.40 | 9.00 |
| 30 | 9.23 | 9.30 | 9.31 | 9.61 | 9.02 |
| 40 | 9.32 | 9.42 | 9.43 | 9.83 | 9.04 |
| 50 | 9.41 | 9.53 | 9.55 | 10.0 | 9.06 |
| 60 | 9.50 | 9.65 | 9.67 | 10.3 | 9.08 |
| 70 | 9.59 | 9.76 | 9.78 | 10.5 | 9.10 |
| 80 | 9.68 | 9.87 | 9.90 | 10.7 | 9.12 |
| 90 | 9.77 | 9.99 | 10.0 | 10.9 | 9.14 |
| 100 | 9.86 | 10.1 | 10.1 | 11.1 | 9.16 |
| 150 | 9.97 | 10.4 | 10.9 | 11.7 | --- |
| 200 | 10.1 | 10.8 | 11.7 | 12.3 | --- |
| 250 | 10.1 | 11.4 | 12.3 | 12.7 | --- |
| 300 | 10.2 | 11.9 | 12.9 | 13.1 | --- |

Table 4. Solubility Parameters of Random Copolymers of Styrene and Substituted Styrene (Part 2)

| Degree of Substitution (%) | Solubility Parameter [(cal/cm³)^{1/2}] | | | | |
|---|---|---|---|---|---|
| | PS-co-Me-CO-PS | PS-co-Et-CO-PS | PS-co-Pr-CO-PS | PS-co-Bu-CO-PS | PS-co-Pen-CO-PS |
| 0 | 8.96 | 8.96 | 8.96 | 8.96 | 8.96 |
| 10 | 9.01 | 9.01 | 9.01 | 9.00 | 8.97 |
| 20 | 9.07 | 9.07 | 9.07 | 9.05 | 8.98 |
| 30 | 9.12 | 9.12 | 9.12 | 9.09 | 8.99 |
| 40 | 9.17 | 9.17 | 9.17 | 9.13 | 9.00 |
| 50 | 9.22 | 9.22 | 9.22 | 9.18 | 9.01 |
| 60 | 9.28 | 9.28 | 9.28 | 9.22 | 9.02 |
| 70 | 9.33 | 9.33 | 9.33 | 9.26 | 9.03 |
| 80 | 9.38 | 9.38 | 9.38 | 9.31 | 9.04 |
| 90 | 9.43 | 9.43 | 9.44 | 9.35 | 9.05 |
| 100 | 9.49 | 9.49 | 9.49 | 9.39 | 9.06 |

Table 5. Solubility Parameters of Random Copolymers of Styrene and Substituted Styrene (Part 3)

| Degree of Substitution (%) | Solubility Parameter [(cal/cm³)^{1/2}] | | | | |
|---|---|---|---|---|---|
| | PS-co-Me-COO-PS | PS-co-Bu-COO-PS | PS-co-Hex-COO-PS | PS-co-Ph-COO-PS | PS-co-Ben-COO-PS |
| 0 | 8.96 | 8.96 | 8.96 | 8.96 | 8.96 |
| 10 | 9.03 | 9.02 | 9.00 | 9.07 | 9.11 |
| 20 | 9.11 | 9.08 | 9.04 | 9.17 | 9.27 |

(continued)

| Degree of Substitution (%) | Solubility Parameter [(cal/cm$^3$)$^{1/2}$] | | | | |
|---|---|---|---|---|---|
| | PS-co-Me-COO-PS | PS-co-Bu-COO-PS | PS-co-Hex-COO-PS | PS-co-Ph-COO-PS | PS-co-Ben-COO-PS |
| 30 | 9.18 | 9.15 | 9.09 | 9.28 | 9.42 |
| 40 | 9.26 | 9.21 | 9.13 | 9.38 | 9.58 |
| 50 | 9.33 | 9.27 | 9.17 | 9.49 | 9.73 |
| 60 | 9.41 | 9.33 | 9.21 | 9.60 | 9.89 |
| 70 | 9.48 | 9.39 | 9.25 | 9.70 | 10.04 |
| 80 | 9.56 | 9.46 | 9.29 | 9.81 | 10.20 |
| 90 | 9.63 | 9.52 | 9.34 | 9.92 | 10.35 |
| 100 | 9.71 | 9.58 | 9.38 | 10.02 | 10.51 |
| 150 | 9.73 | 9.62 | 9.42 | 10.02 | 10.52 |
| 200 | 9.76 | 9.67 | 9.46 | 10.03 | 10.53 |
| 250 | 9.78 | 9.69 | 9.48 | 10.03 | 10.53 |
| 300 | 9.79 | 9.71 | 9.50 | 10.03 | 10.54 |

**Claims**

1. An absorbent article comprising:

   a) a topsheet;
   b) a backsheet joined with the topsheet;
   c) an absorbent core interposed between the topsheet and backsheet; and
   d) an article element;
   wherein the article element comprises a stretch laminate comprising an elastic member comprising a block copolymer comprising at least one soft block and at least two hard blocks;
   wherein the soft block backbone is hydrogenated; and
   wherein the block copolymer comprises one or both of the following:

   a) one or more hard blocks comprising substituted polystyrene;
   b) one or more soft blocks comprising substituted polystyrene,

   wherein the substituted polystyrene has a degree of substitution of greater than 10% and less than 100%; and wherein the substituted polystyrene is selected from a group comprising nitro substituted polystyrene, chlorine substituted polystyrene, bromine substituted polystyrene, nitrile substituted polystyrene, methyl ketone substituted polystyrene, ethyl ketone substituted polystyrene, propyl ketone substituted polystyrene, butyl ketone substituted polystyrene, methyl acetylester substituted polystyrene, ethyl acetylester substituted polystyrene, propyl acetylester substituted polystyrene, butyl acetylester substituted polystyrene, pentyl acetylester substituted polystyrene, hexyl acetylester substituted polystyrene, and combinations thereof.

2. The absorbent article of any of the preceding claims, wherein the one or more hard blocks comprising substituted polystyrene exhibit a solubility parameter of greater than 9.1 (cal/cm$^3$)$^{1/2}$.

3. The absorbent article of any of the preceding claims, wherein the stretch laminate is a slow recovery stretch laminate exhibiting an unload force at 37°C of about 0.16 N/(g/m) or greater and a percent of initial strain after 15 seconds of recovery at 22°C of 10% or greater.

4. The absorbent article of claim 3, wherein the slow recovery stretch laminate exhibits a percent of initial strain after 15 seconds of recovery at 37°C, wherein the difference of the percent of initial strain after 15 seconds of recovery at 22°C and the percent of initial strain after 15 seconds of recovery at 37°C is greater than about 5%.

5. The absorbent article of any of the preceding claims, wherein at least one of the elastic member or the stretch laminate comprises:

    a) 20% to 100% of at least one block copolymer elastomer comprising at least one soft block and at least two hard blocks; wherein the soft block backbone is hydrogenated; wherein the block copolymer comprises one or both of the following:

        (i) one or more hard blocks comprising substituted polystyrene;
        (ii) one or more soft blocks comprising substituted polystyrene;

    b) optionally, 0.01% to 60% of at least one modifying resin; and
    c) optionally, 0.01% to 60% of at least one additive.

6. The absorbent article of the preceding claim, wherein the modifying resin is selected from a group comprising unhydrogenated C5 hydrocarbon resins or C9 hydrocarbon resins, partially and fully hydrogenated C5 hydrocarbon resins or C9 hydrocarbon resins; cycloaliphatic resins; terpene resins; polystyrene and styrene oligomers; poly(t-butylstyrene) or oligomers thereof; rosin and rosin derivatives; coumarone indenes; polycyclopentadiene and oligomers thereof; polymethylstyrene or oligomers thereof; phenolic resins; indene polymers, oligomers and copolymers; acrylate and methacrylate oligomers, polymers, or copolymers; and combinations thereof.

7. The absorbent article of any of the preceding claims, wherein the elastic member is in a form selected from a group comprising a film, a strand, a band, a cross-hatch array, a foam, and combinations thereof.

8. The absorbent article of any of the preceding claims, wherein the absorbent article is selected from a group comprising diapers, training pants, pull-on garments, refastenable pants, adult incontinence products, or feminine care products.

**Patentansprüche**

1. Absorptionsartikel, der Folgendes umfasst:

    a) eine Oberschicht,
    b) eine mit der Oberschicht verbundene Unterschicht,
    c) einen absorbierenden Kern zwischen der Oberschicht und der Unterschicht und
    d) ein Artikelelement,
    wobei das Artikelelement ein Strecklaminat umfasst, welches ein elastisches Element umfasst, welches ein Blockcopolymer umfasst,
    welches mindestens einen Weichblock und zwei Hartblöcke umfasst, wobei das Weichblockgrundgerüst hydriert ist und
    wobei das Blockcopolymer eines oder beide der folgenden umfasst:

        a) einen oder mehrere Hartblöcke, die substituiertes Polystyrol umfassen,
        b) einen oder mehrere Weichblöcke, die substituiertes Polystyrol
        umfassen,

    wobei das substituierte Polystyrol einen Substitutionsgrad von mehr als 10 % und weniger als 100 % aufweist, und
    wobei das substituierte Polystyrol ausgewählt ist aus einer Gruppe umfassend nitrosubstituiertes Polystyrol, chlorsubstituiertes Polystyrol, bromsubstituiertes Polystyrol, nitrilsubstituiertes Polystyrol, methylketonsubstituiertes Polystyrol, ethylketonsubstituiertes Polystyrol, propylketonsubstituiertes Polystyrol, butylketonsubstituiertes Polystyrol, methylacetylestersubstituiertes Polystyrol, ethylacetylestersubstituiertes Polystyrol, propylacetylestersubstituiertes Polystyrol, butylacetylestersubstituiertes Polystyrol, pentylacetylestersubstituiertes Polystyrol, hexylacetylestersubstituiertes Polystyrol und Kombinationen davon.

2. Absorptionsartikel nach einem der vorstehenden Ansprüche, worin der eine oder mehrere Hartblöcke, die substituiertes Polystyrol umfassen, einen Löslichkeitsparameter von größer als 9.1 $(cal/cm^3)^{1/2}$ aufweist.

**3.** Absorptionsartikel nach einem der vorstehenden Ansprüche, worin das Strecklaminat ein sich langsam erholendes Strecklaminat ist, welches eine Entladungskraft von etwa 0,16 N/(g/m) oder größer bei 37 °C und einen Prozentsatz der Anfangsspannung nach 15 Sekunden der Erholung bei 22 °C von 10 % oder größer aufweist.

**4.** Absorptionsartikel nach Anspruch 3, wobei das sich langsam erholende Strecklaminat einen Prozentsatz der Anfangsspannung nach 15 Sekunden der Erholung bei 37 °C aufweist, wobei der Unterschied des Prozentsatzes der Anfangsspannung nach 15 Sekunden der Erholung bei 22 °C und dem Prozentsatz der Anfangsspannung nach 15 Sekunden der Erholung bei 37 °C größer ist als etwa 5 %.

**5.** Absorptionsartikel nach einem der vorstehenden Ansprüche, worin mindestens eines der elastischen Elemente oder das Strecklaminat umfassen:

a) zu 20 % bis 100 % mindestens ein Blockcopolymer-Elastomer, das mindestens einen Weichblock und mindestens zwei Hartblöcke umfasst, wobei das Weichblockgrundgerüst hydriert ist, wobei das Blockcopolymer eines oder beide der folgenden umfasst:

(I) einen oder mehrere Hartblöcke, die substituiertes Polystyrol umfassen,
(II) einen oder mehrere Weichblöcke, die substituiertes Polystyrol umfassen,

b) wahlweise zu 0,01 % bis 60 % mindestens ein modifiziertes Harz
und
c) wahlweise zu 0,01 % bis 60 % mindestens einen Zusatzstoff.

**6.** Absorptionsartikel nach dem vorstehenden Anspruch, wobei das modifizierte Harz ausgewählt ist aus einer Gruppe umfassend unlrydrierte C5-Kohlenwasserstoffharze oder C9-Kohlenwasserstoffharze, teilweise und vollständig hydrierte C5-Kohlenwasserstoffharze oder C9-Kohlenwasserstoffharze, cycloaliphatische Harze, Terpenharze, Polystyrol und Styrololigomere, Poly(t-butylstyrol) oder Oligomere davon, Kolophonium und Kolophoniumderivate, Benzofuran-Inden-Harze, Polycyclopentadien und Oligomere davon, Polymethylstyrol oder Oligomere davon, Phenolharze, Indenpolymere, -oligomere und -copolymere, Acrylat- und Methacrylatoligomere, -polymere, oder -copolymere und Kombinationen davon.

**7.** Absorptionsartikel nach einem der vorstehenden Ansprüche, worin das elastische Element in einer Form vorliegt, die ausgewählt ist aus einer Gruppe umfassend einen Film, einen Strang, ein Band, eine Kreuzschraffuranordnung, einen Schaum und Kombinationen davon.

**8.** Absorptionsartikel nach einem der vorstehenden Ansprüche, worin der Absorptionsartikel ausgewählt ist aus einer Gruppe umfassend Windeln, Übungshosen, Überziehkleidungsstücken, wiederverschließbaren Hosen, Inkontinenzprodukten für Erwachsene oder Damenhygieneprodukten.

**Revendications**

**1.** Article absorbant comprenant :

a) une feuille de dessus ;
b) une feuille de fond jointe à la feuille de dessus ;
c) une âme absorbante intercalée entre la feuille de dessus et la feuille de fond ; et
d) un élément d'article ;
dans lequel l'élément d'article comprend un stratifié étirable comprenant un élément élastique comprenant un copolymère séquencé comprenant au moins un bloc mou et au moins deux blocs durs ;
dans lequel le squelette du bloc mou est hydrogéné ; et
dans lequel le copolymère séquencé comprend l'un et/ou l'autre des blocs suivants :

a) un ou plusieurs blocs durs comprenant du polystyrène substitué ;
b) un ou plusieurs blocs mous comprenant du polystyrène substitué.
dans lequel le polystyrène a un degré de substitution supérieur à 10 % et inférieur à 100 % ; et
dans lequel le polystyrène substitué est choisi parmi un groupe comprenant du polystyrène à substitution nitro, du polystyrène à substitution chlore, du polystyrène à substitution brome, du polystyrène à substitution nitrile, du polystyrène à substitution méthyl-cétone, du polystyrène à substitution éthyl-cétone, du polystyrène à substitution propyl-cétone, du polystyrène à substitution butyl-cétone, du polystyrène à substitution méthyl-acétylester, du polystyrène à substitution éthyl-acétylester, du polystyrène à substitution propyl-acétylester, du polystyrène à substitution butyl-acétylester, du polystyrène à substitution pentyl-acétylester, du polystyrène à substitution hexyl-acétylester, et leurs combinaisons.

2. Article absorbant selon l'une quelconque des revendications précédentes, dans lequel le ou les blocs durs comprenant du polystyrène substitué présentent un paramètre de solubilité supérieur à 9,1 $(cal/cm^3)^{1/2}$.

3. Article absorbant selon l'une quelconque des revendications précédentes, dans lequel le stratifié étirable est un stratifié étirable à rétablissement lent présentant une force à vide à 37 °C d'environ 0,16 N/(g/m) ou plus et un pourcentage de déformation initiale après 15 secondes de rétablissement à 22 °C de 10 % ou plus.

4. Article absorbant selon la revendication 3, dans lequel le stratifié étirable à rétablissement lent présente un pourcentage de déformation initiale après 15 secondes de rétablissement à 37 °C, dans lequel la différence du pourcentage de déformation initiale après 15 secondes de rétablissement à 22 °C et le pourcentage de déformation initiale après 15 secondes de rétablissement à 37 °C est supérieur à environ 5 %.

5. Article absorbant selon l'une quelconque des revendications précédentes, dans lequel au moins l'un parmi l'élément élastique ou le stratifié étirable comprend :

   a) 20 % à 100 % d'au moins un élastomère à copolymère séquencé comprenant au moins un bloc mou et au moins deux blocs durs ; dans lequel le squelette du bloc mou est hydrogéné ; dans lequel le copolymère séquencé comprend l'un et/ou l'autre des blocs suivants :

   (i) un ou plusieurs blocs durs comprenant du polystyrène substitué ;
   (ii) un ou plusieurs blocs mous comprenant du polystyrène substitué ;

   b) facultativement, 0,01 % à 60 % d'au moins une résine de modification ; et
   c) facultativement, 0,01 % à 60 % d'au moins un additif.

6. Article absorbant selon l'une des revendications précédentes, dans lequel la résine de modification est choisie dans un groupe comprenant des résines hydrocarbures en C5 ou résines hydrocarbures en C9 non hydrogénées, des résines hydrocarbures en C5 ou résines hydrocarbures en C9 partiellement et complètement hydrogénées ; des résines cyclo-aliphatiques ; des résines de terpène ; des oligomères de polystyrène et de styrène ; du poly(t-butylstyrène) ou des oligomères de celui-ci ; de la rosine et des dérivés de rosine ; des coumarone indènes ; du polycyclopentadiène et des oligomères de celui-ci ; du polyméthylstyrène ou des oligomères de celui-ci ; des résines phénoliques ; des polymères, oligomères et copolymères d'indène ; des oligomères, polymères ou copolymères d'acrylate et méthacrylate ; et leurs combinaisons.

7. Article absorbant selon l'une quelconque des revendications précédentes, dans lequel l'élément élastique est sous une forme choisie dans un groupe comprenant un film, un brin, une bande, un motif quadrillé, une mousse, et leurs combinaisons.

8. Article absorbant selon l'une quelconque des revendications précédentes, où l'article absorbant est choisi parmi un groupe comprenant des couches, des culottes d'apprentissage à la propreté, des vêtements à enfiler, des culottes refermables, des produits pour l'incontinence chez l'adulte ou des produits d'hygiène féminine.

FIG. 1A

FIG. 1B

FIG. 1C

FIG. 1D

FIG. 1E

FIG. 2

FIG. 3a

FIG. 3b

FIG. 3c

FIG. 4a

FIG. 4b

FIG. 4c

FIG. 5a

FIG. 5b

FIG. 5c

FIG. 6

FIG. 7

FIG. 8

FIG. 9

FIG. 10

FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7717893 B
- US 20050273071 A
- US 20060155255 A
- US 20060167434 A
- US 20090134049 A
- US 5540976 A
- US 20040013852 A
- US 5226992 A
- US 4981747 A
- US 4965122 A
- US 5336545 A
- US 5114781 A
- US 5116662 A
- US 5167897 A
- US 5156793 A

- US 20030088228 A1
- US 20030091807 A1
- US 20040222553 A1
- US 6310154 B
- US 413483 A
- US 11413545 B
- US 676755 P
- US 60676275 P
- US 20050171499 A
- US 20070191806 A
- US 20040162538 A
- US 20050095942 A
- US 5934470 A
- US 5036978 A
- US 5050742 A
- US 5054619 A

### Non-patent literature cited in the description

- Thermoplastic Elastomers. Hanser Publishers, 1996
- Polymer Handbook. Wiley Interscience, 519-559

- **L.H. SPERLING.** Introduction to Physical Polymer Science. Wiley-Interscience, 1992